# EUROPÄISCHE PATENTSCHRIFT

(11) **EP 0 721 933 B1**
(45) Veröffentlichungstag und Bekanntmachung des Hinweises auf die Patenterteilung: **07.07.1999**
(21) Anmeldenummer: 96103784.3
(22) Anmeldetag: 06.12.1990
(51) Int. Cl.: C07C 69/76, C09K 19/20, C07C 69/74, C09K 19/30

(54) **1,4-Disubstituierte 2,6-Difluorbenzolverbindungen und flüssigkristallines Medium**
1,4-Disubstituted 2,6-difluorobenzene compounds and liquid crystal media
Composés 1,4-disubstitués de 2,6-difluorobenzène et milieu cristallin liquide

(30) Priorität: 06.12.1989 DE 3940343
(43) Veröffentlichungstag der Anmeldung: 17.07.1996
(62) Teilanmeldung aus: 91900202.2
(73) Patentinhaber: MERCK PATENT GmbH, 64293 Darmstadt (DE)
(72) Erfinder: Poetsch, Eike Dr., 64367 Mühltal (DE); Meyer, Volker, 64846 Gross-Zimmern (DE); Reiffenrath, Volker, 64380 Rossdorf (DE); Wächtler, Andreas Dr., 72076 Tübingen (DE); Finkenzeller, Ulrich Dr., 68723 Plankstadt (DE); Kurmeier, Hans-Adolf Dr., 64342 Seeheim-Jugenheim (DE); Hittich, Reinhard Dr., 64397 Modautal (DE); Rieger, Bernhard Dr., 64839 Münster (DE); Coates, David Dr., Wimborne, Dorset BH17 7YA (GB); Greenfield, Simon Dr., Poole, Dorset, BH17 7YA (GB); Clemitson, Robert William, Poole, Dorset BH17 9QF (GB)

(56) Entgegenhaltungen:
- EP-A- 0 090 183
- EP-A- 0 387 032
- WO-A-89/08102
- GB-A- 2 232 156
- MOLECULAR CRYSTALS AND LIQUID CRYSTALS (INC. NONLINEAR OPTICS ), Bd. 172, Juli 1989, Seiten 165-189, XP000050356 GRAY G W ET AL: "THE SYNTHESIS AND TRANSITION TEMPERATURES OF SOME FLUORO-SUBSTITUTED 4-CYANOPHENYL AND 4-CYANOBIPHENYL-4'-YL 4-PENTYL- AND 4-BUTOXY-BENZOATES"
- LIQUID CRYSTALS, Bd. 6, Nr. 5, 1.Januar 1989, Seiten 515-523, XP000405976 MCDONELL D G ET AL: "DIPOLE MOMENTS AND DIELECTRIC PROPERTIES OF FLUORINE SUBSTITUTED NEMATIC LIQUID CRYSTALS"

## Beschreibung

Die Erfindung betrifft neue 1,4-disubstituierte 2,6-Difluorbenzolverbindungen der Formel I, 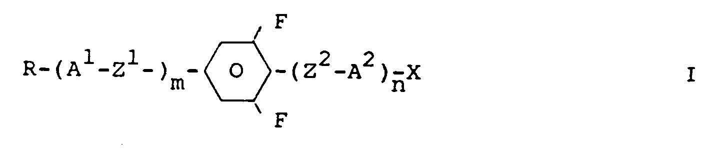 worin
- R: einen unsubstituierten oder einen mindestens einfach durch Halogen substituierten Alkyl- oder Alkenylrest mit 1 bis 15 C-Atomen, wobei in diesen Resten auch eine oder mehrere CH₂-Gruppen jeweils unabhängig voneinander durch -O-, -S-, 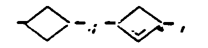 -CO-, -CO-O-, -O-CO- oder -O-CO-O- so ersetzt sein können, daß O-Atome nicht direkt miteinander verknüpft sind,
- A¹ und A²: jeweils unabhängig voneinander einen
(a) trans-1,4-Cyclohexylenrest, worin auch eine oder mehrere nicht benachbarte CH₂-Gruppen durch -O- und/oder -S- ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) und (b) mit CN oder ein- oder zweifach mit Fluor substituiert sein können,
- Z¹ und Z²: jeweils unabhängig voneinander -CO-O-, -O-CO-, -CH₂O-, -OCH₂-, -CH₂CH₂-, -CH=CH-, -C≡C- oder eine Einfachbindung,
- m und n: jeweils unabhängig voneinander 0, 1, 2 oder 3, wobei
- (m+n): 1, 2 oder 3 ist, und
- X: eine der Bedeutungen von R hat oder F, Cl, -CF₃, -OCF₃- -OCF₂H, -OC₂F₅, -CNoder -NCS bedeutet,
mit der Maßgabe, daß mindestens einer der im Molekül vorhandenen Reste Z¹ und Z² -CO-O-, -O-CO-, -CH₂-O-, -OCH₂-, -CH₂CH₂-, -CH=CH- oder -C≡C bedeutet und/oder mindestens einer der im Molekül vorhandenen Reste A¹ und A² 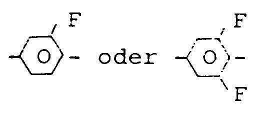 und/oder n 1 oder 2 bedeutet, wobei im Falle -(Z²-A²)ₙ = 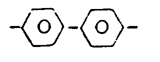 X F, Cl, -CF₃, -OCF₃, -OCF₂H, -OC₂F₅, -CN oder -NCS bedeutet.

Die Erfindung betrifft weiterhin die Verwendung dieser Verbindungen als Komponenten flüssigkristalliner Medien sowie Flüssigkristall- und elektrooptische Anzeige, die die erfindungsgemäßen flüssigkristallinen Medien enthalten.

Die Verbindungen der Formel I können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Medien geeignet sind und insbesondere gleichzeitig eine vergleichsweise geringe Viskosität besitzen sowie eine relativ hohe dielektrische Anisotropie.

Es wurde nun gefunden, daß Verbindungen der Formel I als Komponenten flüssigkristalliner Medien vorzüglich geeignet sind. Insbesondere verfügen sie über vergleichsweise niedere Viskositäten. Mit ihrer Hilfe lassen sich stabile flüssigkristalline Medien mit breitem Mesophasenbereich und vorteilhaften Werten für die optische und dielektrische Anisotropie sowie mit hervorragender UV- und Temperaturstabilität erhalten.

Flüssigkristalle der Formel 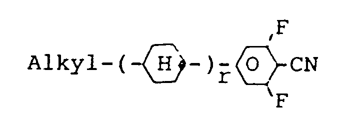 r = 1 oder 2
sind bereits aus DE 3209178 bekannt. Aus der JP 62-103057 sind Verbindungen der Formeln 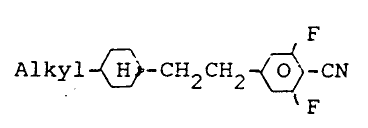 und 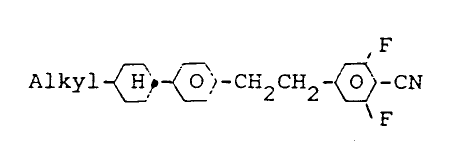 bekannt. In JP 63-216858 schließlich werden Verbindungen der Formel 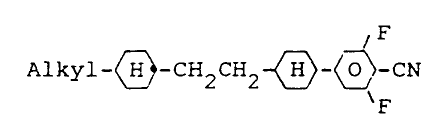 beschrieben. Im Hinblick auf die verschiedensten Einsatzbereiche derartiger Verbindungen mit sehr hohem Δε war es jedoch wünschenswert, weitere Verbindungen zur Verfügung zu haben, die auf die jeweiligen Anwendungen genau maßgeschneiderte Eigenschaften aufweisen.

Flüssigkristalle der Formel 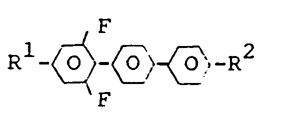 sind bereits aus WO 89/02425 bekannt und zeichnen sich durch eine hohe Doppelbrechung und relativ hohe Viskosität aus.

Im Hinblick auf die verschiedensten Einsatzbereiche derartiger Verbindungen mit positivem Δε war es wünschenswert, weitere Verbindungen zur Verfügung zu haben, die auf die jeweiligen Anwendungen genau maßgeschneiderte Eigenschaften aufweisen.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Verbindungen der Formel I sowie die Verwendung dieser Verbindungen als Komponenten flüssigkristalliner Medien. Gegenstand der Erfindung sind ferner flüssigkristalline Medien mit einem Gehalt an mindestens einer Verbindung der Formel I sowie Flüssigkristallanzeigeelemente, insbesondere elektroopische Anzeigeelemente, die derartige Medien enthalten.

Der Einfachheit halber bedeuten im folgenden A³ einen Rest der Formel 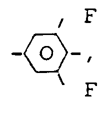 Cyc einen 1,4-Cyclohexylenrest, A⁴ einen Rest der Formel 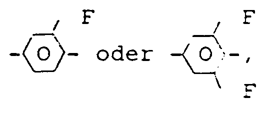 Che einen 1,4-Cyclohexenylenrest, Dio einen 1,3-Dioxan-2,5-diylrest, Dit einen 1,3-Dithian-2,5-diylrest, Phe einen 1,4-Phenylenrest, Pyd einen Pyridin-2,5-diylrest, Pyr einen Pyrimidin-2,5-diylrest und Bi einen Bicyclo(2,2,2)-octylenrest.

Die Verbindungen der Formel I umfassen dementsprechend die bevorzugten Verbindungen mit zwei Ringen der Teilformeln Ia und Ib;

R-A³-A²-X Ia

R-A³-Z²-A²-X Ib

Verbindungen mit drei Ringen der Teilformeln Ic bis If:

R-A³-Z²-A²-Z²-A²-X Ic

R-A¹-Z¹-A³-Z²-A²-X Id

R-A¹-Z¹-A⁴-Z¹-A³-X Ie

R-A⁴-Z¹-A¹-Z¹-A³-X If

sowie Verbindungen mit vier Ringen der Teilformeln Ig bis Ij:

R-A¹-Z¹-A³-Z²-A²-Z²-A²-X Ig

R-A¹-Z¹-A¹-Z¹-A³-Z²-A²-X Ih

R-A¹-Z¹-A¹-Z¹-A⁴-Z¹-A³-X Ii

R-A¹-Z¹-A⁴-Z¹-A¹-Z¹-A³-X Ij

Darunter sind besonders diejenigen der Teilformeln Id, Ie, Ih und Ii bevorzugt.

In den Verbindungen der vor- und nachstehenden Formeln bedeutet X vorzugsweise-OCF₃, -OCHF₂ ,-CF₃, F oder Cl. Ebenfalls bevorzugt sind Verbindungen der vor- und nachstehdnen Formeln, worin X eine der Bedeutungen von R hat.

Im Falle X = F, Cl, -CF₃, -OCF₃, -OCHF₂, -OC₂F₅, -CN oder -NCS ist vorzugsweise mindestens einer der im Molekül der Formel I vorhandenen Reste A¹ und A² 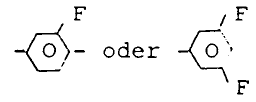 und/oder n
bedeutet 1 oder 2.

R bedeutet vorzugsweise Alkyl, ferner Alkoxy. A¹ und/oder A² bedeuten bevorzugt Phe, Cyc, Che, Pyr oder Dio. Bevorzugt enthalten die Verbindungen der Formel I nicht mehr als einen der Reste Bi, Pyd, Pyr, Dio oder Dit.

Bevorzugt sind auch Verbindungen der Formel I sowie aller Teilformeln, in denen A¹ und/oder A² ein- oder zweifach durch F oder einfach durch CN substituiertes 1,4-Phenylen bedeutet. Insbesondere sind dies 2-Fluor-1,4-phenylen, 3-Fluor-1,4-phenylen und 2,3-Difluor-1,4-phenylen sowie 2-Cyan-1,4-phenylen und 3-Cyan-1,4-phenylen.

Z¹ und Z² bedeuten bevorzugt eine Einfachbindung, -CO-O-, -O-CO- und -CH₂CH₂-, in zweiter Linie bevorzugt -CH₂O- und -OCH₂-. Vorzugsweise ist nur eine der im Molekül vorhandenen Gruppe Z² und Z² von der Einfachbindung verschieden.

Falls R einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R einen Alkylrest bedeutet, in dem eine CH₂-Gruppe durch -CH=CH- ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R einen Alkylrest bedeutet, in dem eine CH₂-Gruppe durch -O- und eine durch -CO- ersetzt ist, so sind diese bevorzugt benachbart. Somit beeinhalten diese eine Acyloxygruppe -CO-O- oder eine Oxycarbonylgruppe -O-CO-. Vorzugsweise sind diese geradkettig und haben 2 bis 6 C-Atome.

Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R einen Alkylrest bedeutet, in dem eine CH₂-Gruppe durch unsubstituiertes oder substituiertes -CH=CH- und eine benachbarte CH₂-Gruppe durch CO oder CO-O oder O-CO-ersetzt ist, so kann dieser geradkettig oder verzweigt sein. Vorzugsweise ist er geradkettig und hat 4 bis 13 C-Atome. Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Verbindungen der Formel I, die über für Polymerisationsreaktionen geeignete Flügelgruppen R verfügen, eignen sich zur Darstellung flüssigkristalliner Polymerer.

Verbindungen der Formeln I mit verzweigten Flügelgruppen R können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verbindungen der Formel I mit S_{A}-Phasen eignen sich beispielsweise für thermisch adressierte Displays.

Verzweigte Gruppen dieser Art enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R einen Alkylrest darstellt, in dem zwei oder mehr CH₂-Gruppen durch -O- und/oder -CO-O- ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxyethyl, 3,3-Bis-carboxy-propyl, 4,4-Bis-carboxy-butyl, 5,5-Bis-carboxy-pentyl, 6,6-Bis-carboxy-hexyl, 7,7-Bis-carboxy-heptyl, 8,8-Bis-carboxy-octyl, 9,9-Bis-carboxynonyl, 10,10-Bis-carboxy-decyl, Bis-(methoxycarbonyl)-methyl, 2,2-Bis-(methoxycarbonyl)-ethyl, 3,3-Bis-(methoxycarbonyl)-propyl, 4,4-Bis-(methoxycarbonyl)-butyl, 5,5-Bis-(methoxycarbonyl)-pentyl, 6,6-Bis-(methoxycarbonyl)-hexyl, 7,7-Bis-(methoxycarbonyl)-heptyl, 8,8-Bis-(methoxycarbonyl)-octyl, Bis-(ethoxycarbonyl)-methyl, 2,2-Bis-(ethoxycarbonyl)-ethyl, 3,3-Bis-(ethoxycarbonyl)-propyl, 4,4-Bis-(ethoxycarbonyl)-butyl, 5,5-Bis-(ethoxycarbonyl)-hexyl.

Verbindungen der Formel I, die über für Polykondensationen geeignete Flügelgruppen R verfügen, eignen sich zur Darstellung flüssigkristalliner Polykondensate.

Formel I umfaßt sowohl die Racemate dieser Verbindungen als auch die optischen Antipoden sowie deren Gemische.

Unter diesen Verbindungen der Formel I sowie den Unterformeln sind diejenigen bevorzugt, in denen mindestens einer der darin enthaltenden Reste eine der angegebenen bevorzugten Bedeutungen hat.

In den Verbindungen der Formel I sind diejenigen Stereoisomeren bevorzugt, in denen die Ringe Cyc und Piperidin trans-1,4-disubstituiert sind. Diejenigen der vorstehend genannten Formeln, die eine oder mehrere Gruppen Pyd, Pyr und/oder Dio enthalten, umschließen jeweils die beiden 2,5-Stellungsisomeren.

Die 1,4-Cyclohexenylen-Gruppe hat vorzugsweise folgende Strukturen:

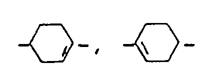

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart Bd IX, S. 867 ff.) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die erfindungsgemäßen Verbindungen können z.B. hergestellt, indem man eine Verbindung der Formel II, 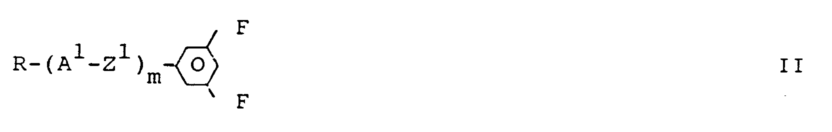 worin R, A¹, Z¹ und m die angegebene Bedeutung haben, gemäß folgenden Reaktionsschemata umsetzt: 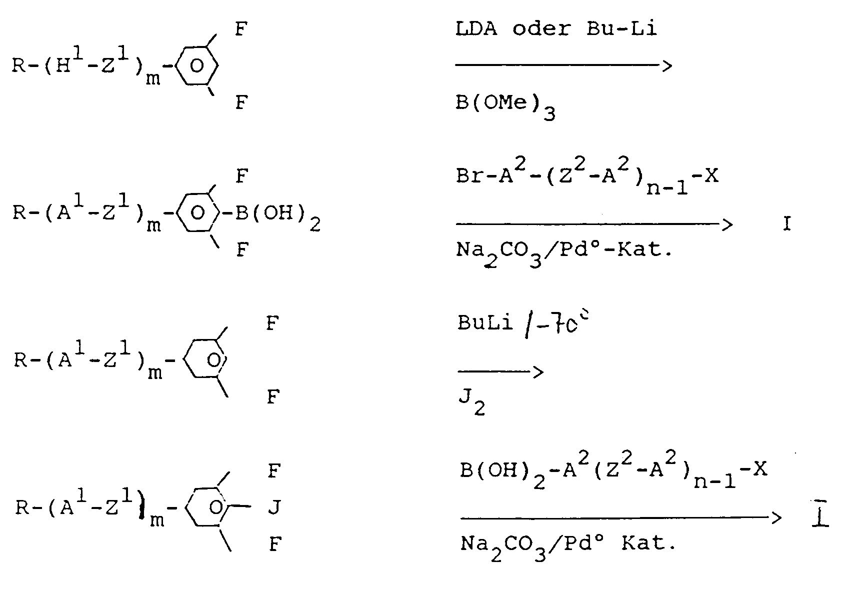 Weitere Synthesemethoden sind für den Fachmann augenscheinlich. Beispielsweise können in 5-Position entsprechend substituierte 1,3-Difluorbenzol-Verbindungen gemäß obigem Schema in die 2-substituierten 1,3-Difluorverbindungen überführt werden und der Rest R-(A¹-Z¹)ₘ-anschließend durch in der Flüssigkristallchemie gebräuchliche Reaktionen (z.B. Veresterung, Veretherung oder Kopplungen z.B. gemäß der Artikel E. Poetsch, Kontakte (Darmstadt) 1988 (2), S. 15) eingeführt werden oder umgekehrt.

Die Verbindungen der Formel II können beispielsweise nach folgenden Syntheseschemata hergestellt werden:

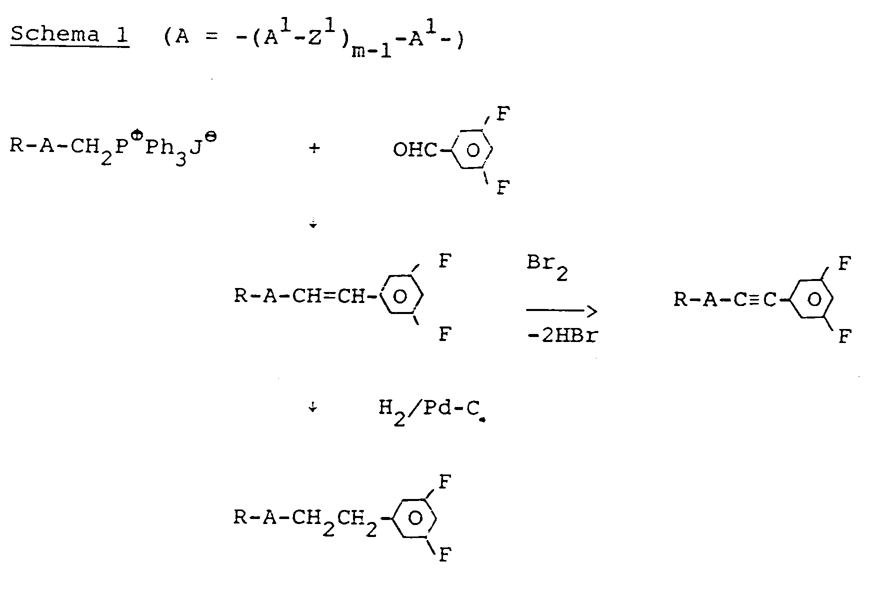

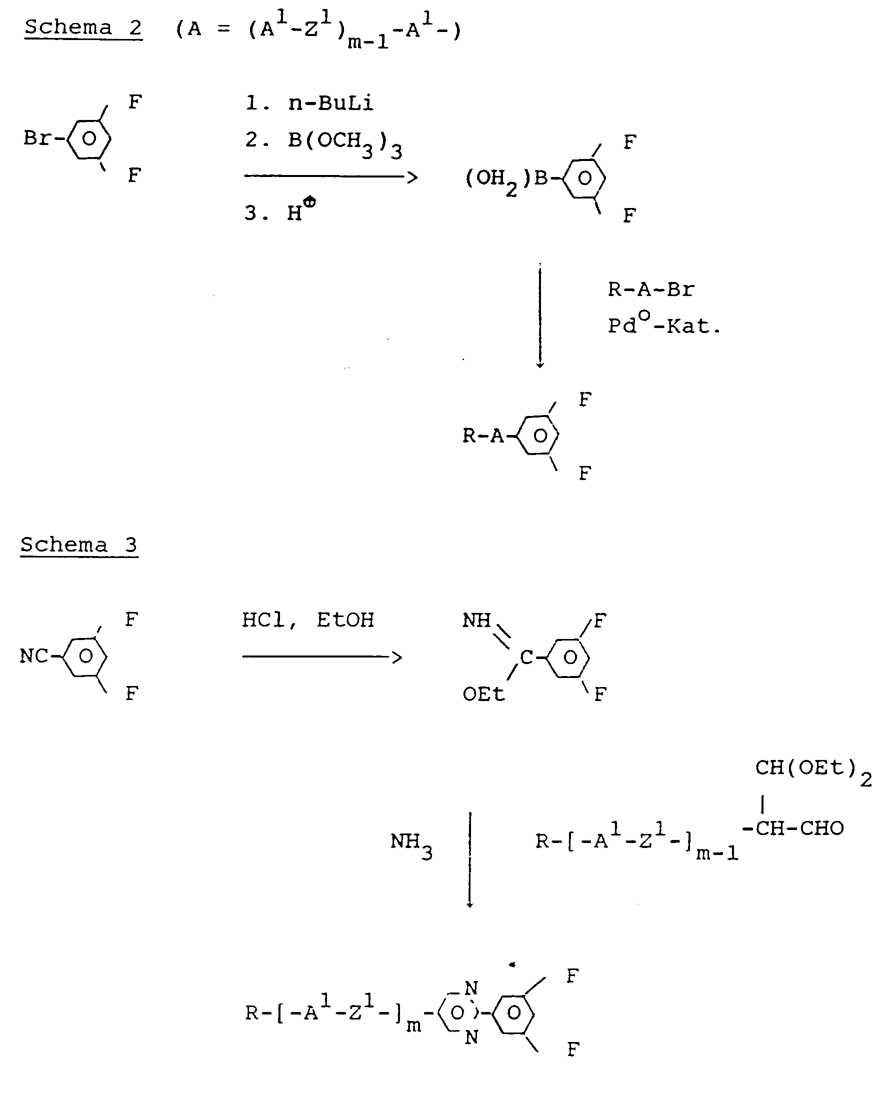

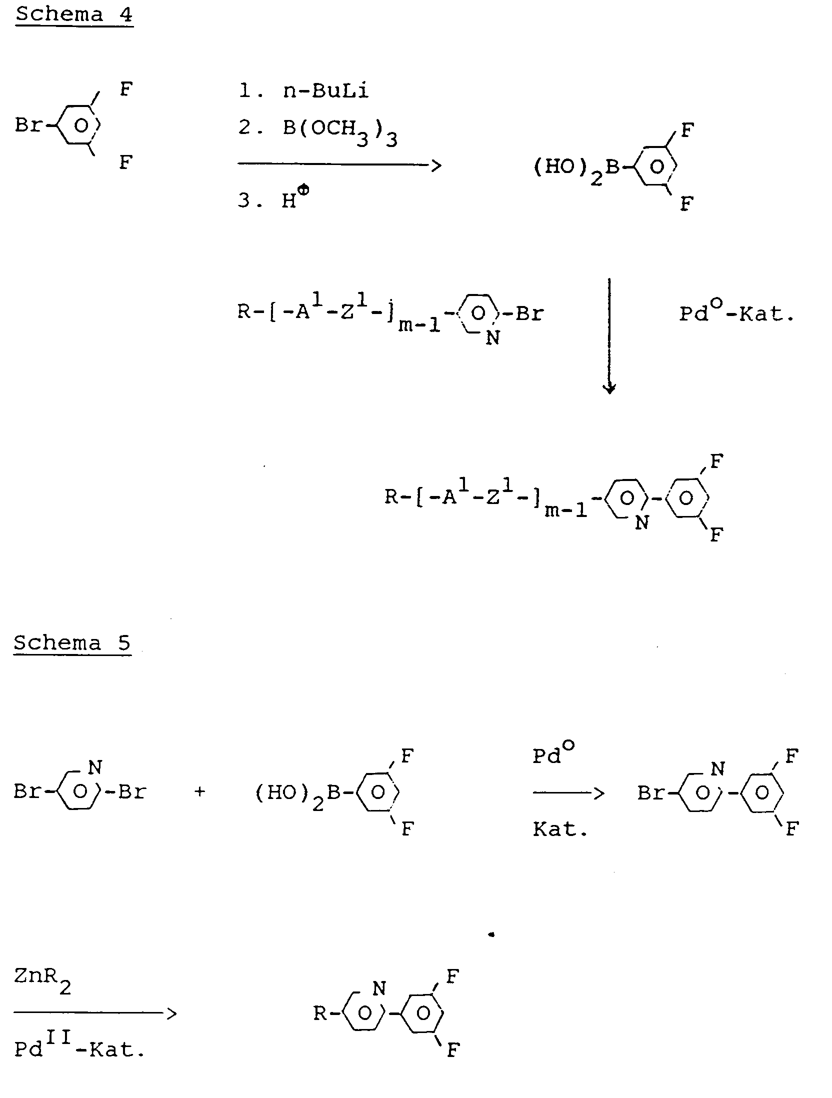

Die Ausgangsmaterialien sind entweder bekannt oder können in Analogie zu bekannten Verbindungen hergestellt werden.

Ester der Formel I können auch durch Veresterung entsprechender Carbonsäuren (oder ihrer reaktionsfähigen Derivate) mit Alkoholen bzw. Phenolen (oder ihren reaktionsfähigen Derivaten) oder nach der DCC-Methode (DCC = Dicyclohexylcarbodiimid) erhalten werden.

Die entsprechenden Carbonsäuren und Alkohole bzw. Phenole sind bekannt oder können in Analogie zu bekannten Verfahren oder auch durch Umsetzung von metalliertem II mit geeigneten Elektrophilen (B(OH)₃/H₂O₂ bzw. CO₂) hergestellt werden.

Die Synthese einiger besonders bevorzugter Verbindungen ist im folgenden näher angegeben (Y = H oder F, Z = H oder F):

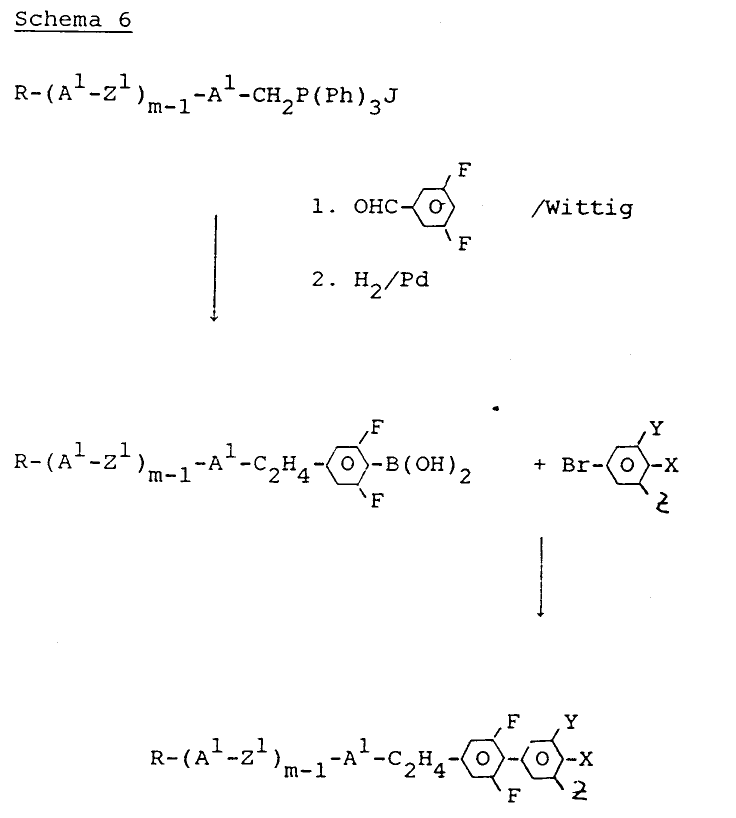

In einem weiteren Verfahren zur Herstellung der Verbindungen der Formel I setzt man ein Arylhalogenid mit einem Olefin um in Gegenwart eines tertiären Amins und eines Palladiumkatalysators (vgl. R.F. Heck, Acc. Chem. Res. 12 (1979) 146). Geeignete Arylhalogenide sind beispielsweise Chloride, Bromide und Iodide, insbesondere Bromide und Iodide. Die für das Gelingen der Kupplungsreaktion erforderlichen tertiären Amine, wie z.B. Triethylamin, eignen sich auch als Lösungsmittel. Als Palladiumkatalysatoren sind beispielsweise dessen Salze, insbesondere Pd(II)-acetat, mit organischen Phosphor(III)-Verbindungen wie z.B. Triarylphosphanen geeignet. Man kann dabei in Gegenwart oder Abwesenheit eines inerten Losungsmittels bei Temperaturen zwischen etwa 0° und 150°, vorzugsweise zwischen 20° und 100°, arbeiten; als Lösungsmittel kommen z.B. Nitrile wie Acetonitril oder Kohlenwasserstoffe wie Benzol oder Toluol in Betracht. Die als Ausgangsstoffe eingesetzten Arylhalogenide und Olefine sind vielfach im Handel erhältlich oder können nach literaturbekannten Verfahren hergestellt werden, beispielsweise durch Halogenierung entsprechender Stammverbindungen bzw. durch Eliminierungsreaktionen an entsprechenden Alkoholen oder Halogeniden.

Auf diese Weise sind beispielsweise Stilbenderivate herstellbar. Die Stilbene können weiterhin hergestellt werden durch Umsetzung eines 4-substituierten Benzaldehyds mit einem entsprechenden Phosphorylid nach Wittig. Man kann aber auch Tolane der Formel I herstellen, indem man anstelle des Olefins monosubstituiertes Acetylen einsetzt (Synthesis 627 (1980) oder Tetrahedron Lett. 27, 1171 (1986)).

Weiterhin können zur Kopplung von Aromaten Arylhalogenide mit Arylzinkverbindungen umgesetzt werden. Bevorzugt werden diese Reaktionen unter Zusatz eines Katalysators wie z.B. eines Palladium(0)komplexes in inerten Lösungsmitteln wie Kohlenwasserstoffen bei hohen Temperaturen, z.B. in siedendem Xylol, unter Schutzgas durchgeführt.

Kopplungen von Alkinyl-Verbindungen mit Arylhalogeniden können analog dem von A.O. King, E. Negishi, F.J. Villani und A. Silveira in J.Org.Chem. 43, 358 (1978) beschriebenen Verfahren durchgeführt werden.

Tolane der Formel I können auch über die Fritsch-Buttenberg-Wiechell-Umlagerung (Ann. 279, 319, 332, 1984) hergestellt werden, bei der 1,1-Diaryl-2-halogenethylene umgelagert werden zu Diarylacetylenen in Gegenwart starker Basen.

Tolane der Formel I können auch hergestellt werden, indem man die entsprechenden Stilbene bromiert und anschließend einer Dehydrohalogenierung unterwirft. Dabei kann man an sich bekannte, hier nicht näher erwähnte Varianten dieser Umsetzung anwenden.

Ether der Formel I sind durch Veretherung entsprechender Hydroxyverbindungen, vorzugsweise entsprechender Phenole, erhüältlich, wobei die Hydroxyverbindung zweckmäßig zunächst in ein entsprechendes Metallderivat, z.B. durch Behandeln mit NaH, NaNH₂, NaOH, KOH, Na₂CO₃ oder K₂CO₃ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel, wie z.B. Aceton, 1,2-Dimethoxyethan, DMF oder Dimethylsulfoxid oder auch mit einem Überschuß an wäßriger oder wäßrig-alkoholischer NaOH oder KOH bei Temperaturen zwischen etwa 20° und 100°.

Besonders bevorzugt sind Verbindungen der Formel Ia 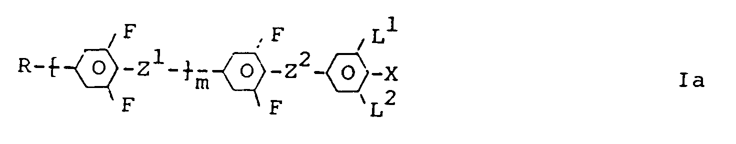 worin R, Z¹, Z² und X die angegebenen Bedeutungen haben, m 1 oder 2 ist und L¹ und L² jeweils unabhängig voneinander H oder F bedeuten. Im Falle m = 2 sind die beiden Reste Z¹ gleich oder verschieden. Vorzugsweise ist mindestens einer der im Molekül der Formel Ia vorhandenen Reste Z¹ und Z² von der Einfachbindung verschieden und bedeutet vorzugsweise -CH₂CH₂-, -C≡C- oder -CO-O-.

Ausgehend von dem käuflichen 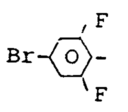 lassen sich die Verbindungen nach an und für sich bekannten Methoden darstellen. Außer den im nachstehenden Schema aufgeführten Verknüpfungsmöglichkeiten ergeben sich auch analoge Syntheseverfahren über Friedel-Crafts-Acylierung oder Wittig-Reaktion mit nachfolgender Reduktion bzw. Hydrierung für den Aufbau der CH₂-CH₂ verknüpften Substanzen.

Die 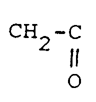 enthaltenden Verbindungen lassen sich über eine Chlorierung mittels PCl₅ zu 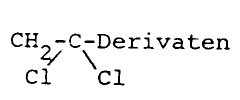 und nachfolgende HCl-Eliminierung mittels Base in die Acetylene umwandeln. Die Transformation der -CH=CH- verbrückten Substanzen, die über Heck-Kopplung oder Wittig-Reaktion herstellbar sind, gelingt nach herkömmlicher Weise z.B. mittels Bromierung und doppelter HBr-Abspaltung.

Die Einführung der Carboxylat-Gruppe gelingt in Analogie zur Einführung der CH₂CH₂-Gruppe (vergl. Verbindung 7, nachstehendes Formelschema) mittels CO₂. Die intermediär gebildeten Säuren lassen sich mit den entsprechenden Phenolen verestern, die aus 1 gemäß Syntheseschritt ba darstellbar sind.

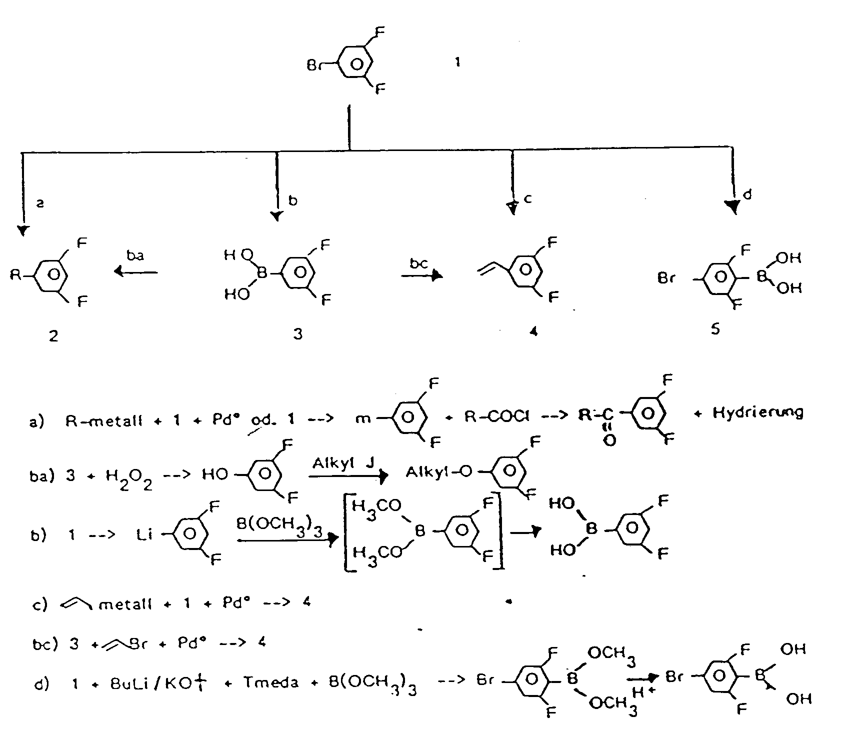

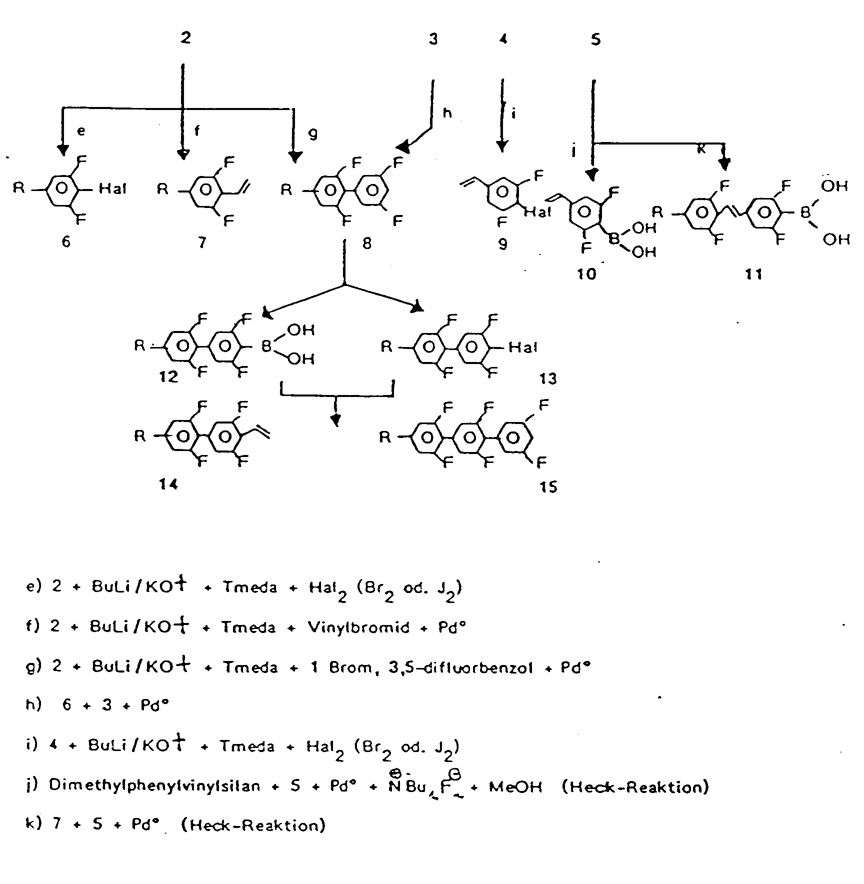

Die Verbindung 8 läßt sich gemäß d) zu 12 und analog e) zu 13 umsetzen. 12 wird mittels Vinylbromid gemäß bc) und 13 mittels Divinylzink gemäß c) zu 14 umgesetzt. 15 läßt sich aus 12 und 1 mittels Kreuzkopplung gemäß h) erhalten oder aus 13 und 3 durch Kreuzkopplung.

Durch geeignete Kombination der Bausteine 1-15 lassen sich alle Vorstufen von Ia aufbauen, in denen Z gleich C≡C oder die Einfachbindung ist. Die CH=CH-Gruppierung ist in die CH₂-CH₂ oder -C≡C-Gruppierung überführbar.

Die Einführung der Endgruppierung 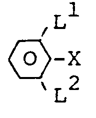 gelingt entweder durch Verwendung eines Kopplungsschrittes mit 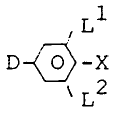 (D = Hal, m, 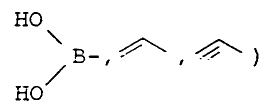 oder durch Einführung von X in Verbindungen mit der Endgruppierung 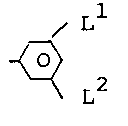 Die Herstellung von ebenfalls die CO₂ und/oder C≡C Zwischenglieder enthaltenden Verbindungen laßt sich sinngemäß nach analogen Formelschemata durchführen.

Weitere Synthesevarianten sind dem Fachmann bekannt und gehen von entsprechend substituierten Brombiphenylen aus. Bevorzugte Varianten sind den folgenden Schemata zu entnehmen. Alle Ausgangsmaterialien sind entweder bekannt oder können in Analogie zu bekannten Verbindungen hergestellt werden.

Diese Brombiphenyle können in an sich bekannter Weise durch edelmetallkatalysierte Kreuzkopplungsreaktionen hergestellt werden (E. Poetsch, Kontakte (Darmstadt) 1988 (2) p. 15).

Eine Variante ist beispielhaft im folgenden angegeben:

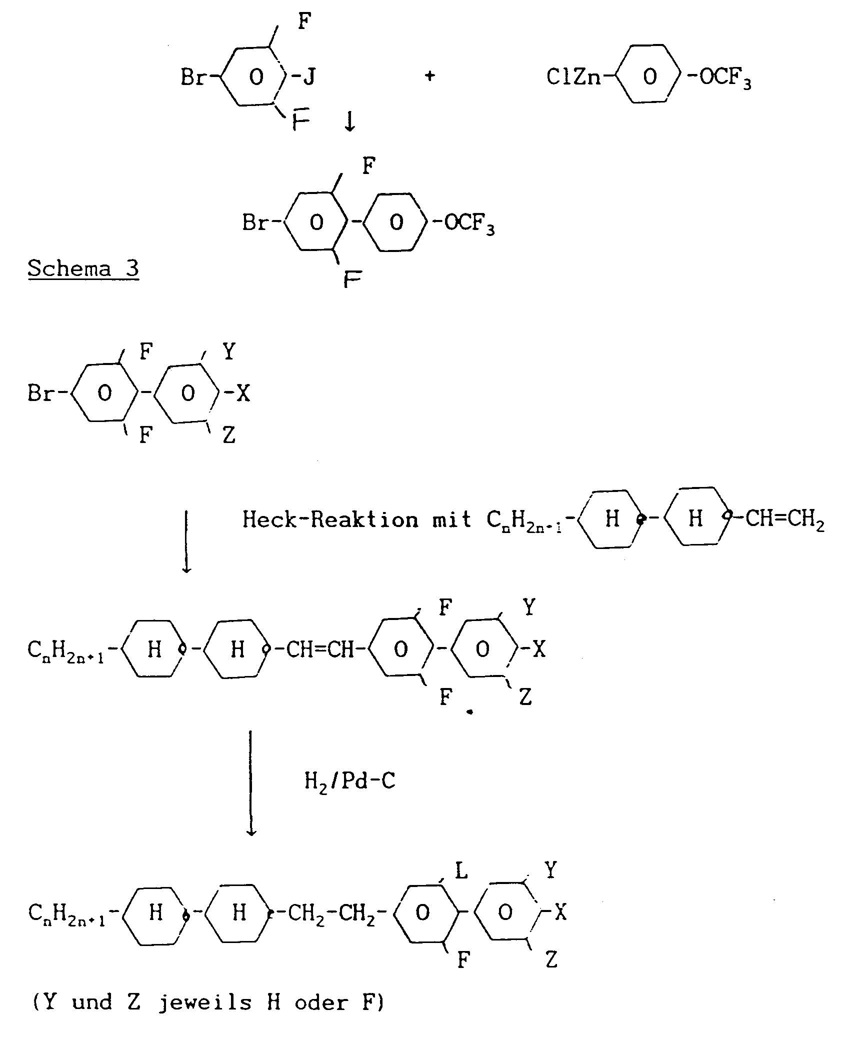

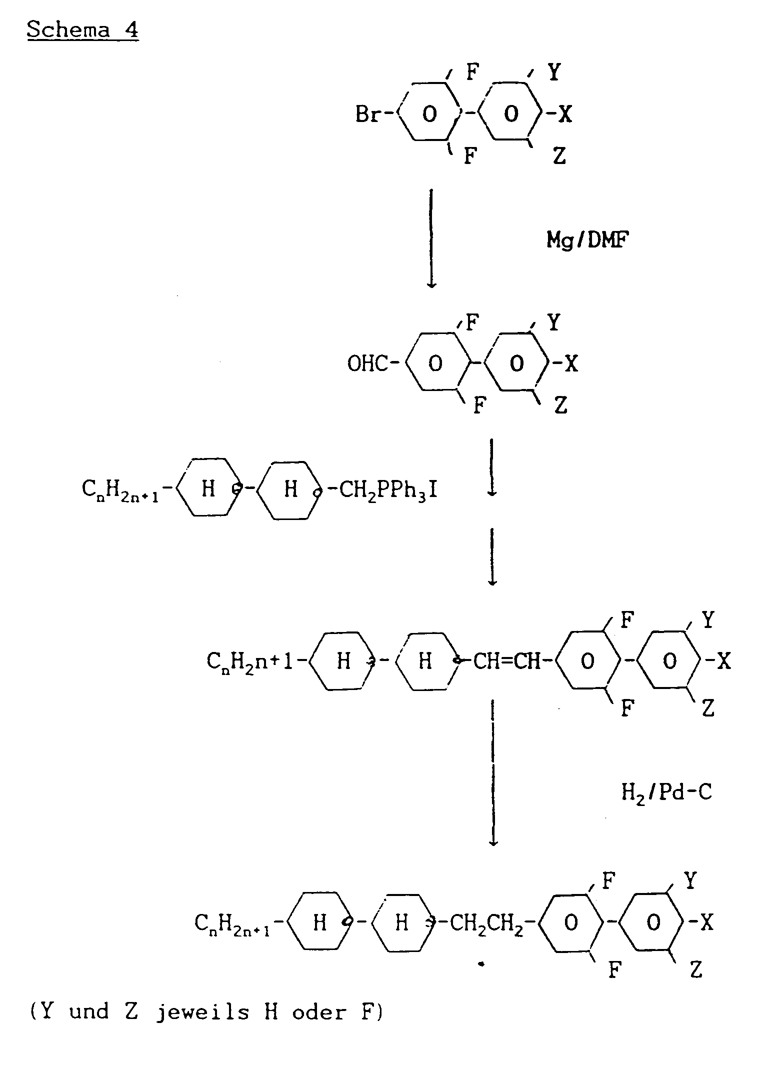

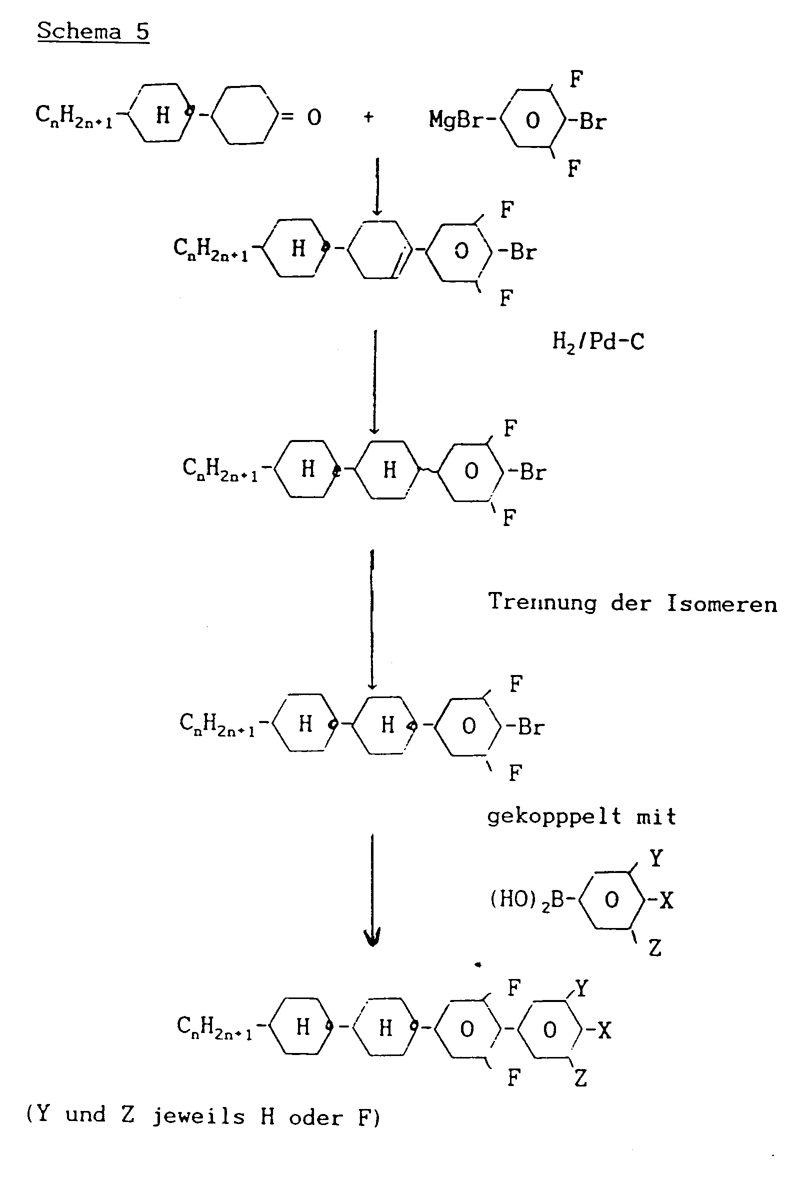

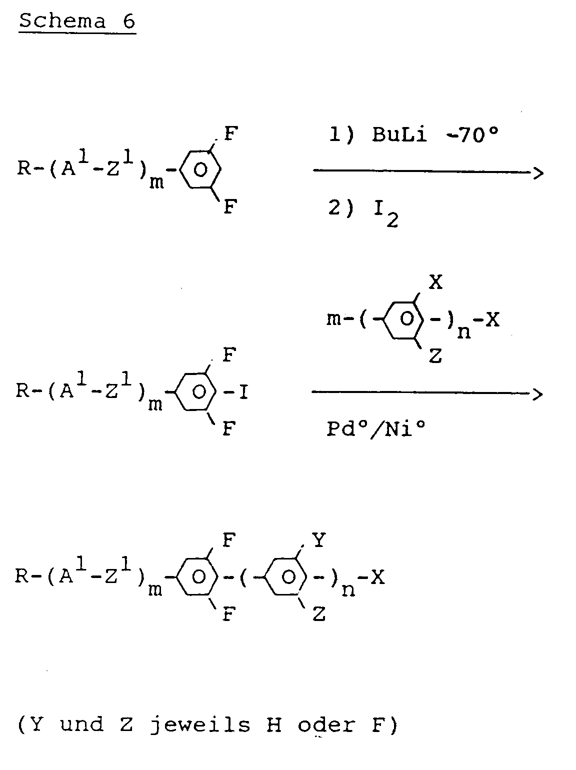

Einige besonders bevorzugte, kleinere Gruppen von Verbindungen der Formel I sind im folgenden angegeben:

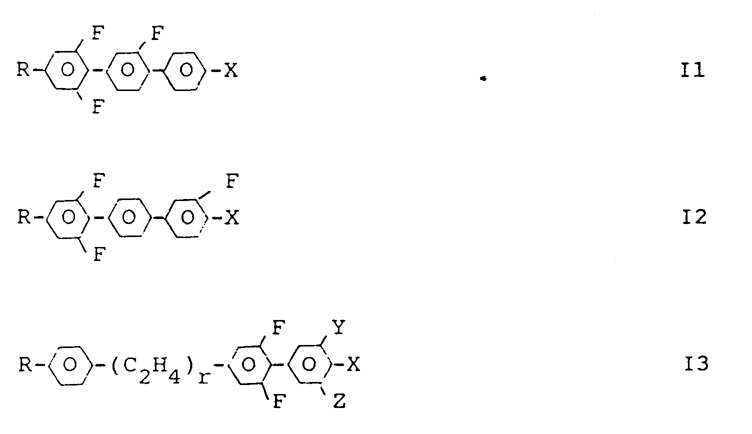

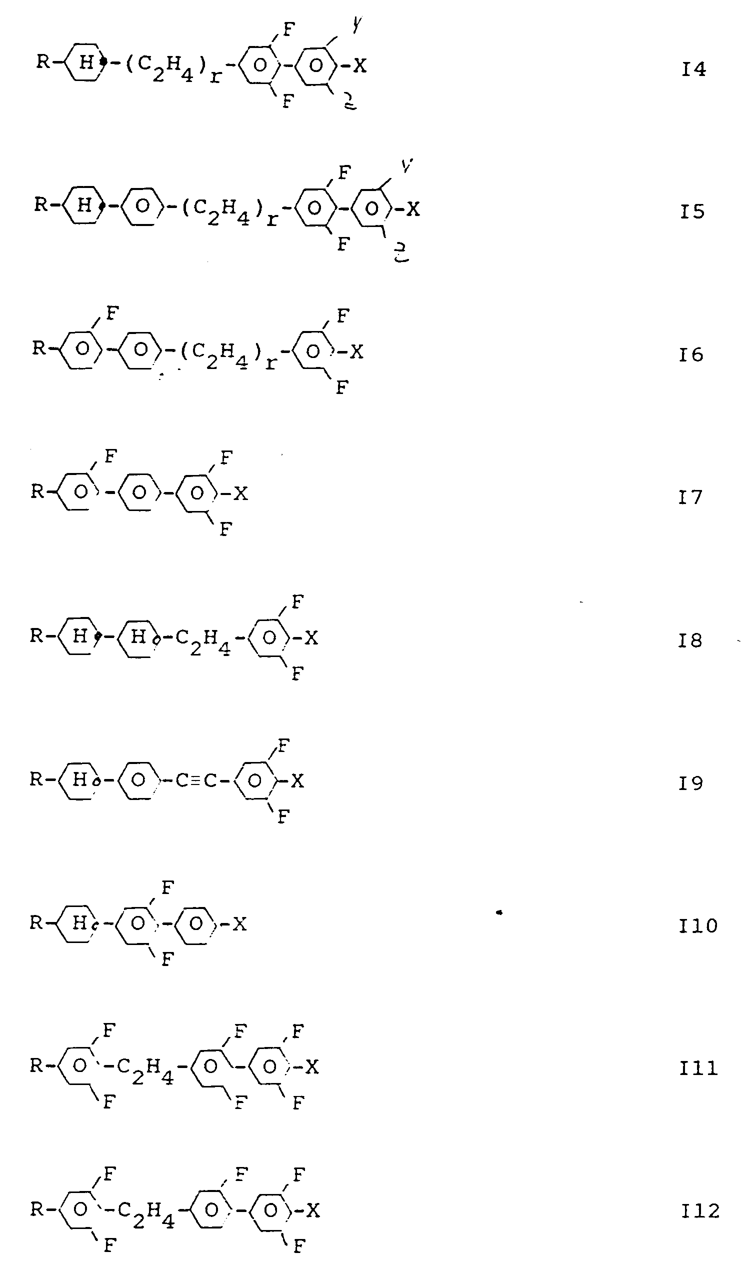

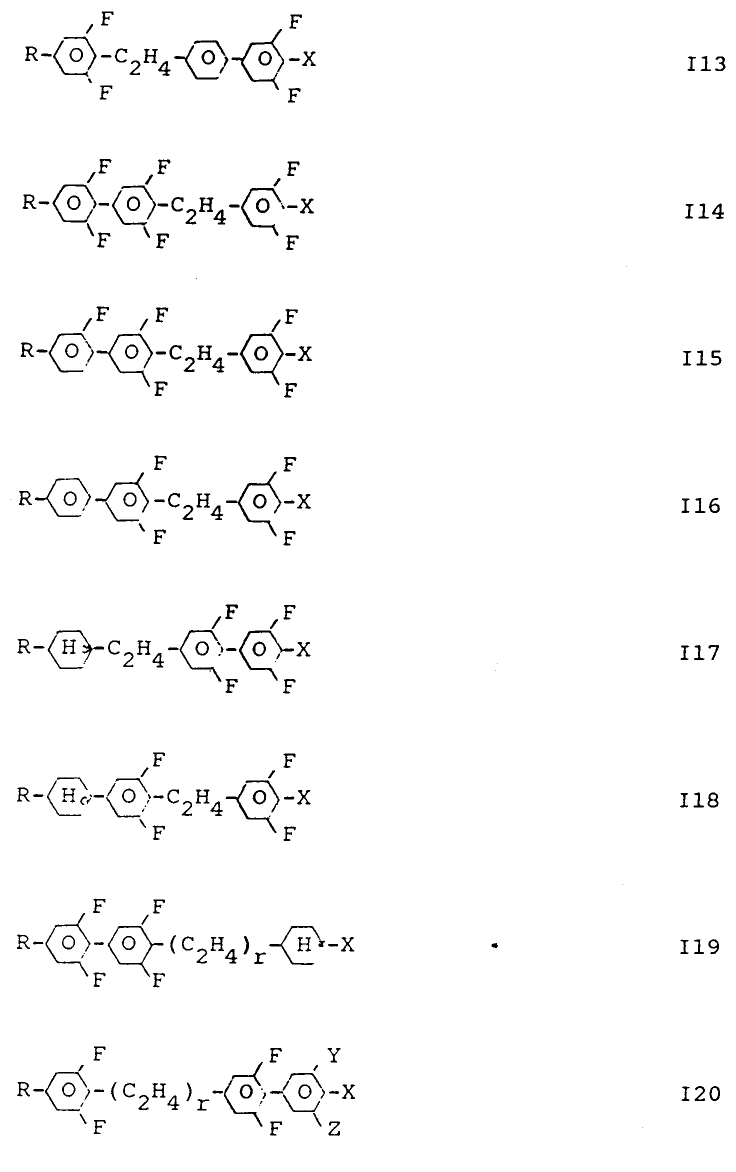

In Formel I8 ist X vorzugsweise R.

Besonders bevorzugt sind auch erfindungsgemäße Verbindungen worin einer der Reste Z¹ und Z² -CH=CH- bedeutet. Diese können z.B. nach folgendem Schema hergestellt werden:

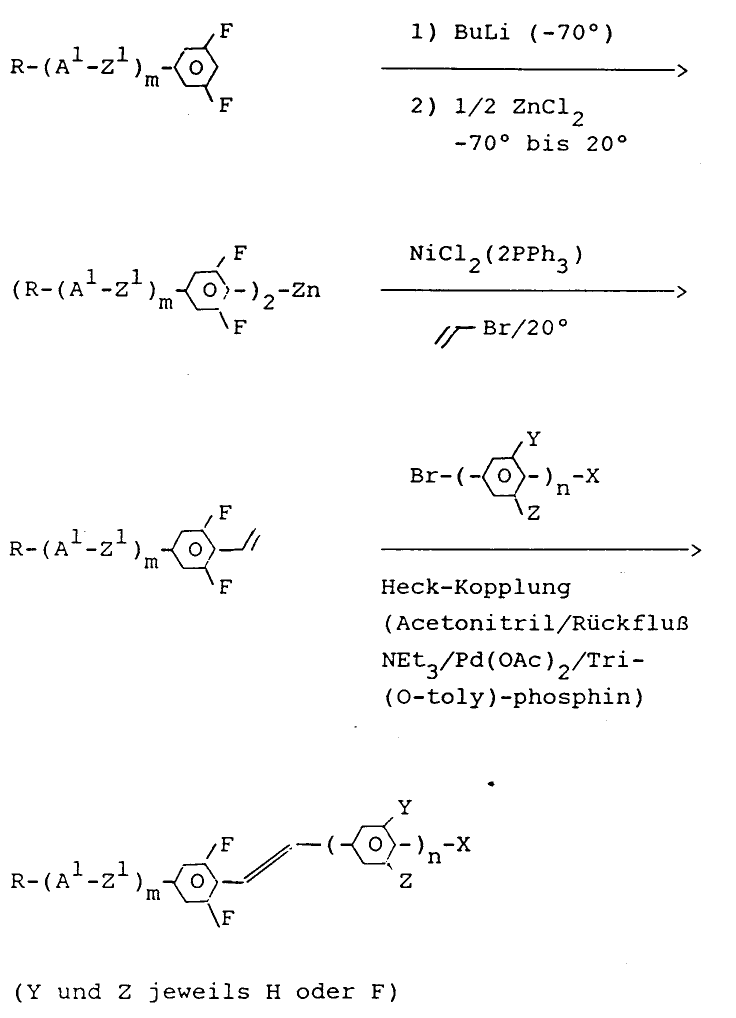

Durch Hydrierung an Pd/C bzw. PtO₂ bei 1-4 bar können auch entsprechende Verbindungen mit -CH₂CH₂- anstelle -CH=CH- erhalten werden.

Besonders bevorzugt sind Verbindungen der Formel I, worin einer der Reste Z¹ und Z² -CO-O-, -O-CO-, -CH₂CH₂-, -CH=CH- oder -C≡C- ist und die anderen Reste Z¹ bzw. Z² eine Einfachbindung bedeuten.

Besonders bevorzugte Tolanderivate können nach folgenden Schemata analog den bekannten Heck-Kopplungen hergestellt werden:

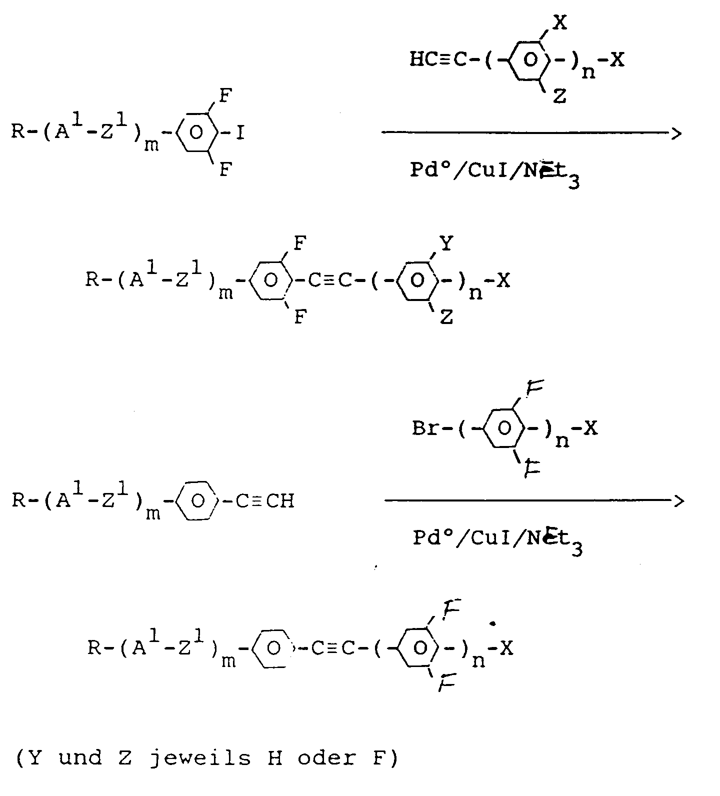

Weitere Details zur Synthese bzw. entsprechender Vorprodukte können den Internationalen Patentanmeldungen PCT/EP 90/01471, PCT/EP 90/01437 und WO 90/08757 entnommen werden.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäurephenyl- oder cyclohexyl-ester, Phenyl- oder Cyclohexylphenyl- oder cyclohexyl-ester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexene, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexylphenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R'' 1

R'-L-COO-E-R'' 2

R'-L-OOC-E-R'' 3

R'-L-CH₂CH₂-E-R'' 4

R'-L-C≡C-E-R'' 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Rest L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R'' bedeuten in den Verbindungen der Teilformeln 1a, 2a, 3a, 4a und 5a jeweils unabhängig voneinander Alkyl, Alkenyl, Alkoxy, Alkenyloxy oder Alkanoyloxy mit bis zu 8 Kohlenstoffatomen. Bei den meisten dieser Verbindungen sind R' und R'' voneinander verschieden, wobei einer dieser Reste meist Alkyl oder Alkenyl ist. In den Verbindungen der Teilformeln 1b, 2b, 3b, 4b und 5b bedeutet R'' -CN, -CF₃, F, Cl oder -NCS; R hat dabei die bei den Verbindungen der Teilformeln 1a bis 5a angegebene Bedeutung und ist vorzugsweise Alkyl oder Alkenyl. Aber auch andere Varianten der vorgesehenen Substituenten in den Verbindungen der Formeln 1, 2, 3, 4 und 5 sind gebräuchlich. Viele solcher Substanzen oder auch Gemische davon sind im Handel erhältlich. Alle diese Substanzen sind nach literaturbekannten Methoden oder in Analogie dazu erhältlich.

Die erfindungsgemäßen Medien enthalten vorzugsweise neben Komponenten aus der Gruppe der Verbindungen 1a, 2a, 3a, 4a und 5a (Gruppe 1) auch Komponenten aus der Gruppe der Verbindungen 1b, 2b, 3b, 4b und 5b (Gruppe 2), deren Anteile vorzugsweise wie folgt sind:
Gruppe 1: 20 bis 90 %, insbesondere 30 bis 90 %,
Gruppe 2: 10 bis 80 %, insbesondere 10 bis 50 %,
   wobei die Summe der Anteile der erfindungsgemäßen Verbindungen und der Verbindungen aus den Gruppen 1 und 2 bis zu 100 % ergeben.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungegemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

Die folgenden Beispiele sollen die Erfindung erläutern, ohne sie zu begrenzen. Vor- und nachstehend bedeuten Potenzangaben Gewichtsprozent. Alle Temperaturen sind in Grad Gelsius angegeben. Fp. bedeutet Schmelzpunkt Kp = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. Δn bedeutet optische Anisotropie (589 nm, 20 °C) und die Viskosität (mm²/sec) wurde bei 20 °C bestimmt.

"Übliche Aufarbeitung" bedeutet: man gibt gegebenenfalls Wasser hinzu, extrahiert mit Methylenchlorid, Diethylether oder Toluol, trennt ab, trocknet die organische Phase, dampft ein und reinigt das Produkt durch Destillation unter reduziertem Druck oder Kristallisation und/oder Chromatographie. Folgende Abkürzungen werden verwendet:
- DAST: Diethylaminoschwefeltrifluorid
- DCC: Dicyclohexylcarbodiimid
- DDQ: Dichlordicyanobenzochinon
- DIBALH: Diisobutylaluminiumhydrid
- KOT: Kalium-tertiär-butanolat
- THF: Tetrahydrofuran
- pTSOH: p-Toluolsulfonsäure
- TMEDA: Tetramethylethylendiamin

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Rest CₙH₂ₙ₊₁ und CₘH₂ₘ₊₁ sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R¹, R², L¹ und L²:

| Code für R¹, R², L¹, L² | R¹ | R² | L¹ | L² |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| nT | CₙH₂ₙ₊₁ | CN | H | H |
| nN.F | CₙH₂ₙ₊₁ | CN | H | F |
| nF | CₙH₂ₙ₊₁ | F | H | H |
| nOF | OCₙH₂ₙ₊₁ | F | H | H |
| nCl | CₙH₂ₙ₊₁ | Cl | H | H |
| nF.F | CₙH₂ₙ₊₁ | F | H | F |
| nOmFF | CₙH₂ₙ₊₁ | OCₘH₂ₘ₊₁ | F | F |
| nmF | CₙH₂ₙ₊₁ | CₘH₂ₘ₊₁ | F | H |
| nCF₃ | CₙH₂ₙ₊₁ | CF₃ | H | H |
| nOCF₃ | CₙH₂ₙ₊₁ | OCF₃ | H | H |
| nOCF₂ | CₙH₂ₙ₊₁ | OCHF₂ | H | H |
| nS | CₙH₂ₙ₊₁ | NCS | H | H |
| rVsN | CᵣH₂ᵣ₊₁-CH=CH-CₛH₂ₛ- | CN | H | H |
| rEsN | CᵣH₂ᵣ₊₁-O-CₛH₂ₛ- | CN | H | H |
| nNF | CₙH₂ₙ₊₁ | CN | F | H |
| nAm | CₙH₂ₙ₊₁ | COOCₘH₂ₘ₊₁ | H | H |

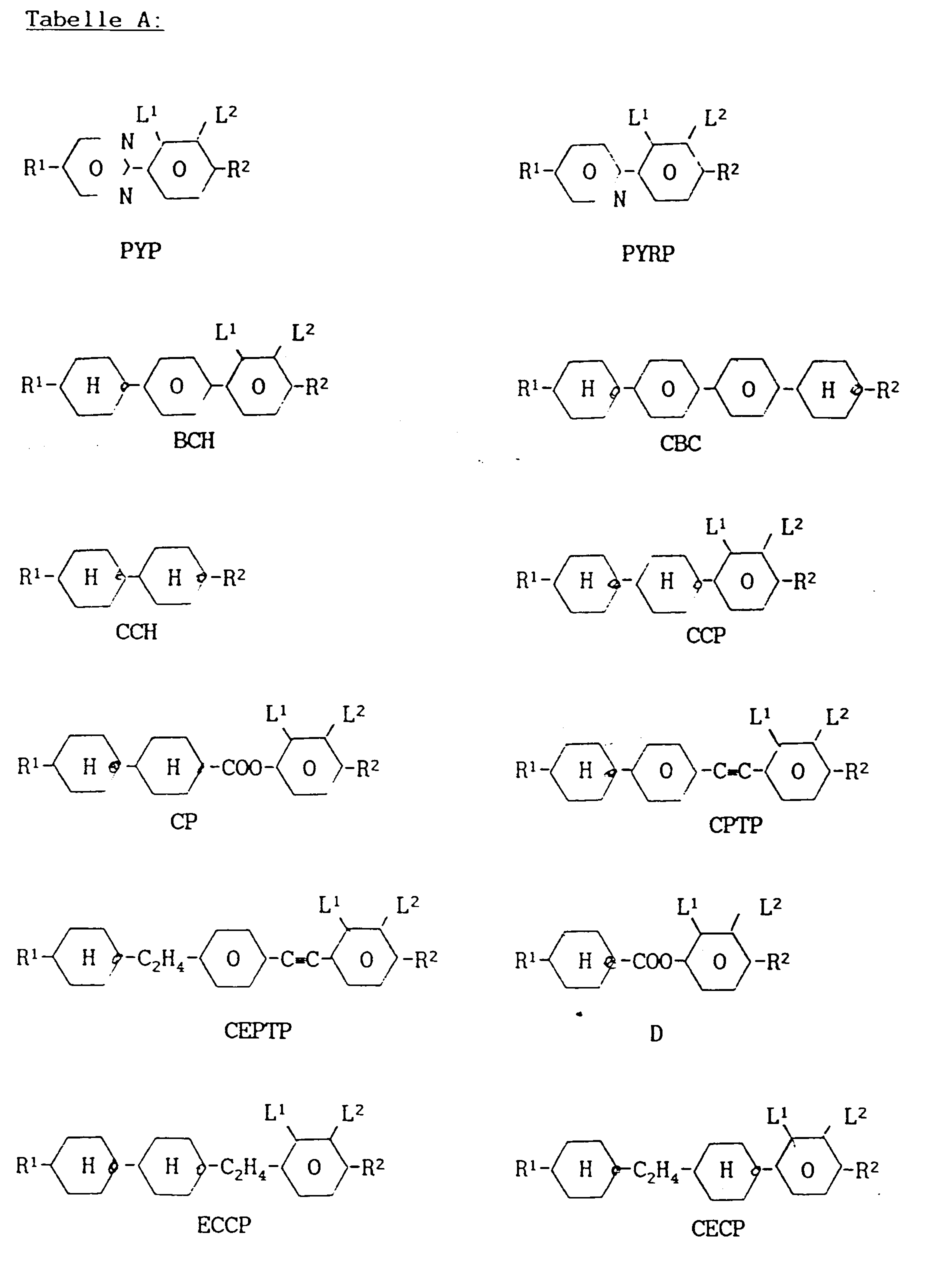

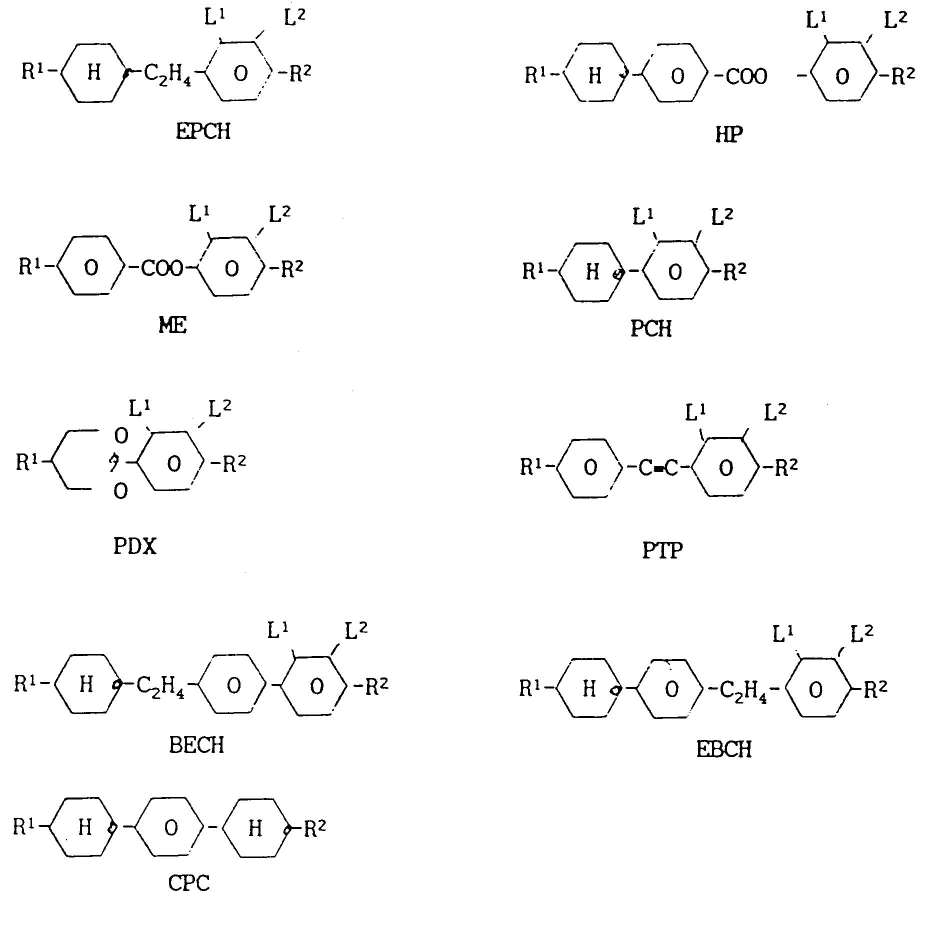

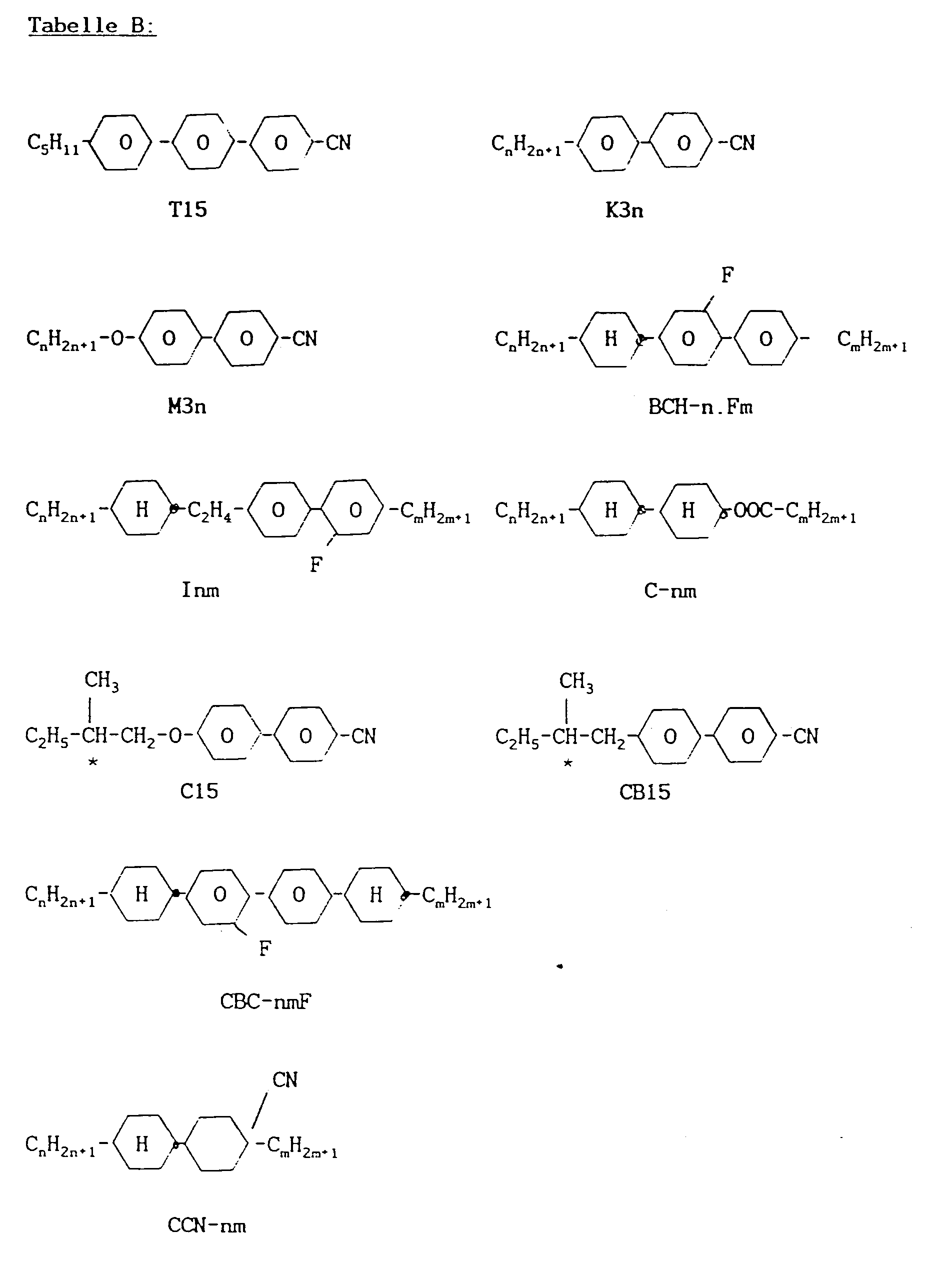

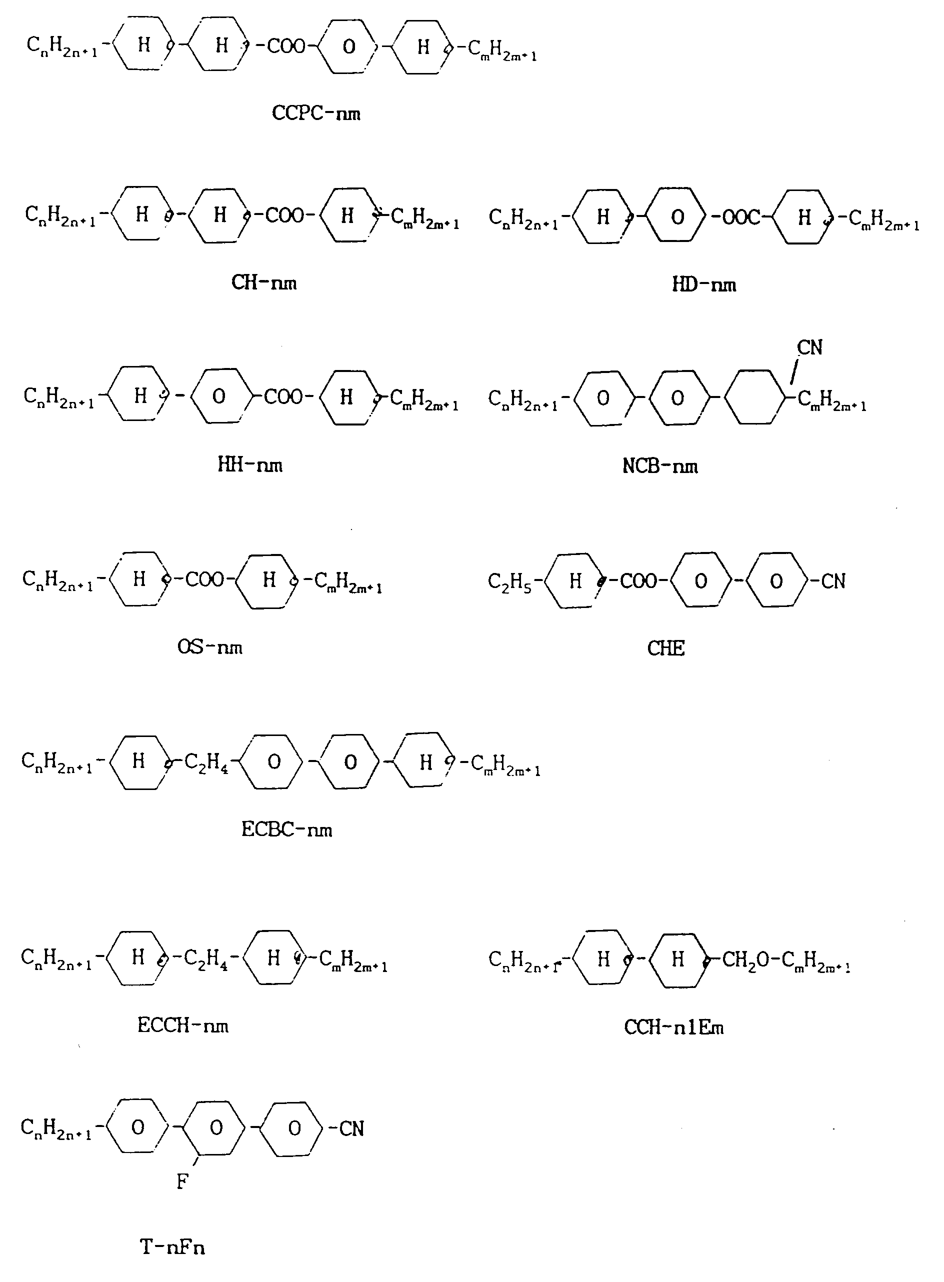

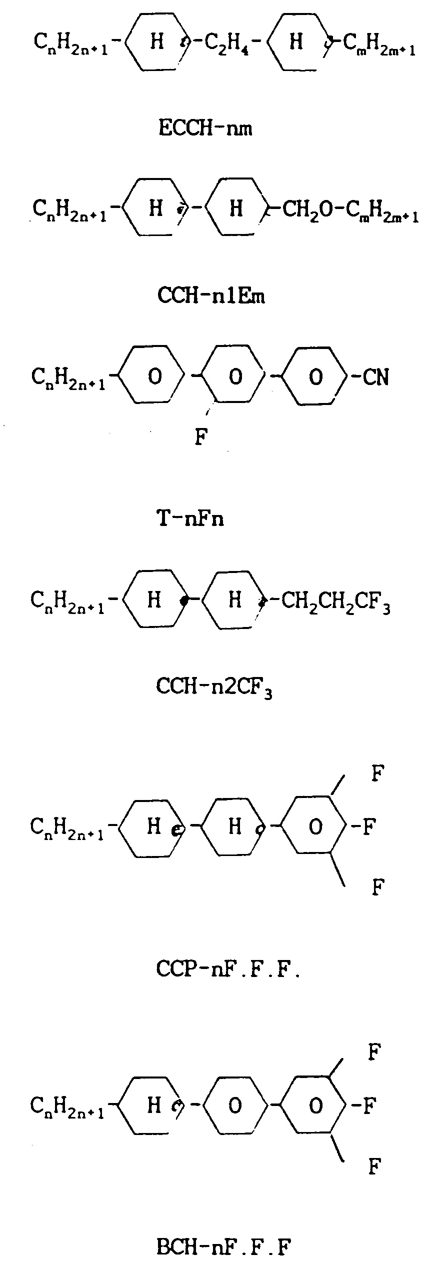

### Beispiel 1

Ein Gemisch von 9,2 g 2,6-Difluor-4-[2-(p-ethoxyphenyl)-ethyl]-phenylboronsäure (hergestellt durch Umsetzung von 3,5-Difluorbenzaldehyd mit p-Ethoxybenzyltriphenylphosphoniumjodid nach Wittig und nachfolgender Hydrierung des erhaltenen Styrolderivates an Pd/C), 5,9 g 1-Brom-3,4-difluorbenzol, 38 ml 2 mol wässeriger Na₂CO₃-Lsg., 0,6 g Tetrakis(triphenylphosphin)palladium-(0) und 75 ml Toluol wird zwei Stunden am Rückfluß gekocht. Die wässerige Phase wird zweimal mit Toluol extrahiert und mit der org. Phase vereinigt. Nach üblicher wässeriger Aufarbeitung und chromatographischer Aufreinigung des Rückstandes erhält man 4-[2-(p-Ethoxyphenyl)-ethyl]-2,6,3',4'-tetrafluorbiphenyl, K 92 I, Δε +24.

### Beispiel 2 bis 58

Analog nach einem der beiden Schemata auf Seite 12 erhält man aus den entsprechenden Vorstufen der Formel II die folgenden erfindungsgemäßen Verbindungen:

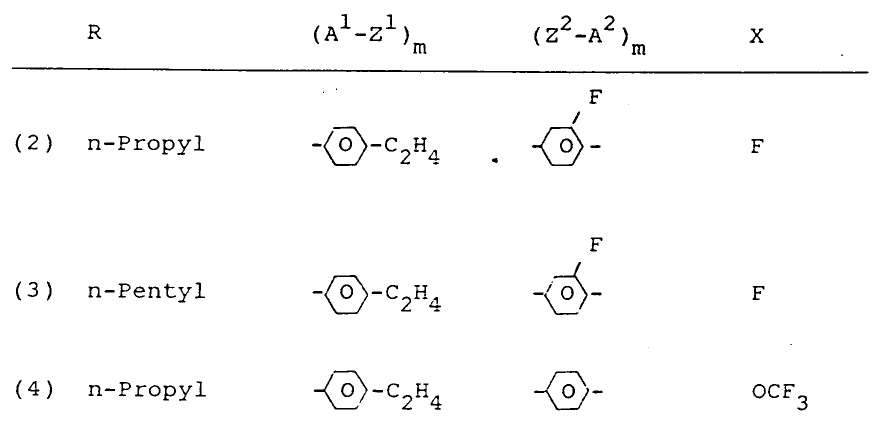

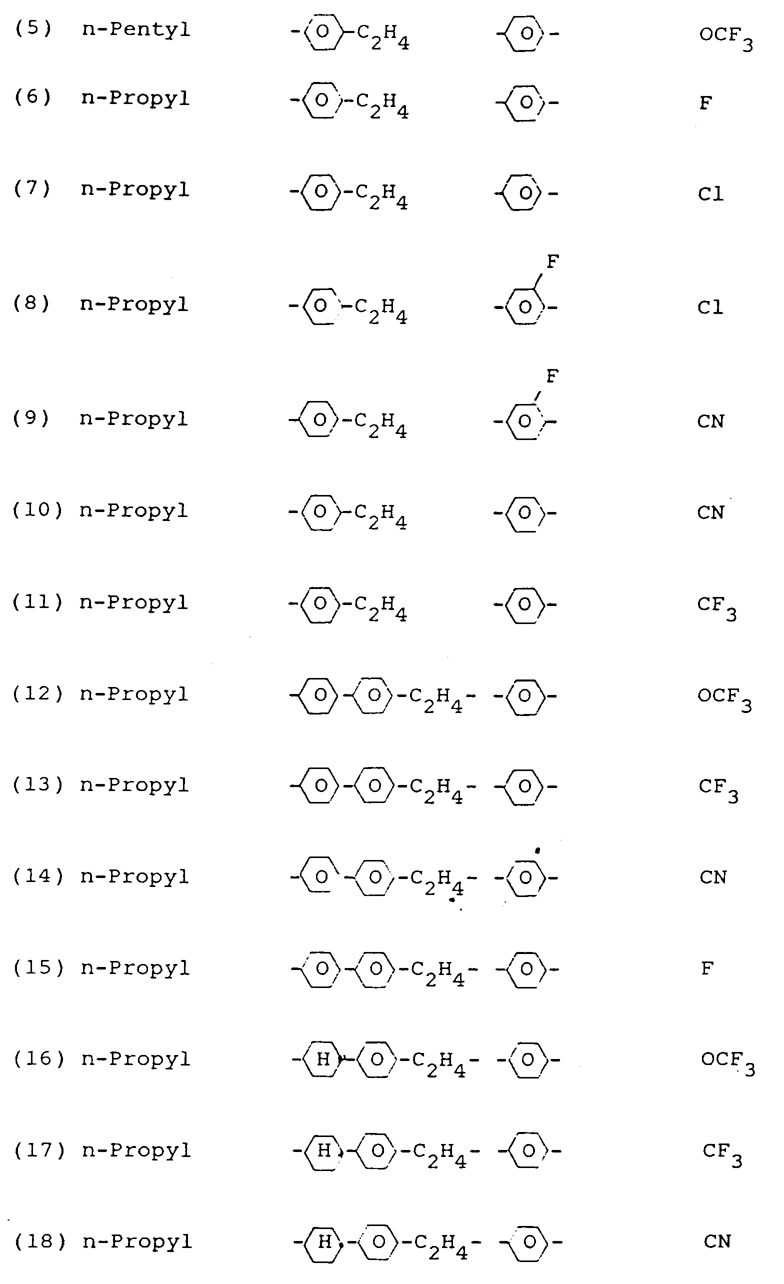

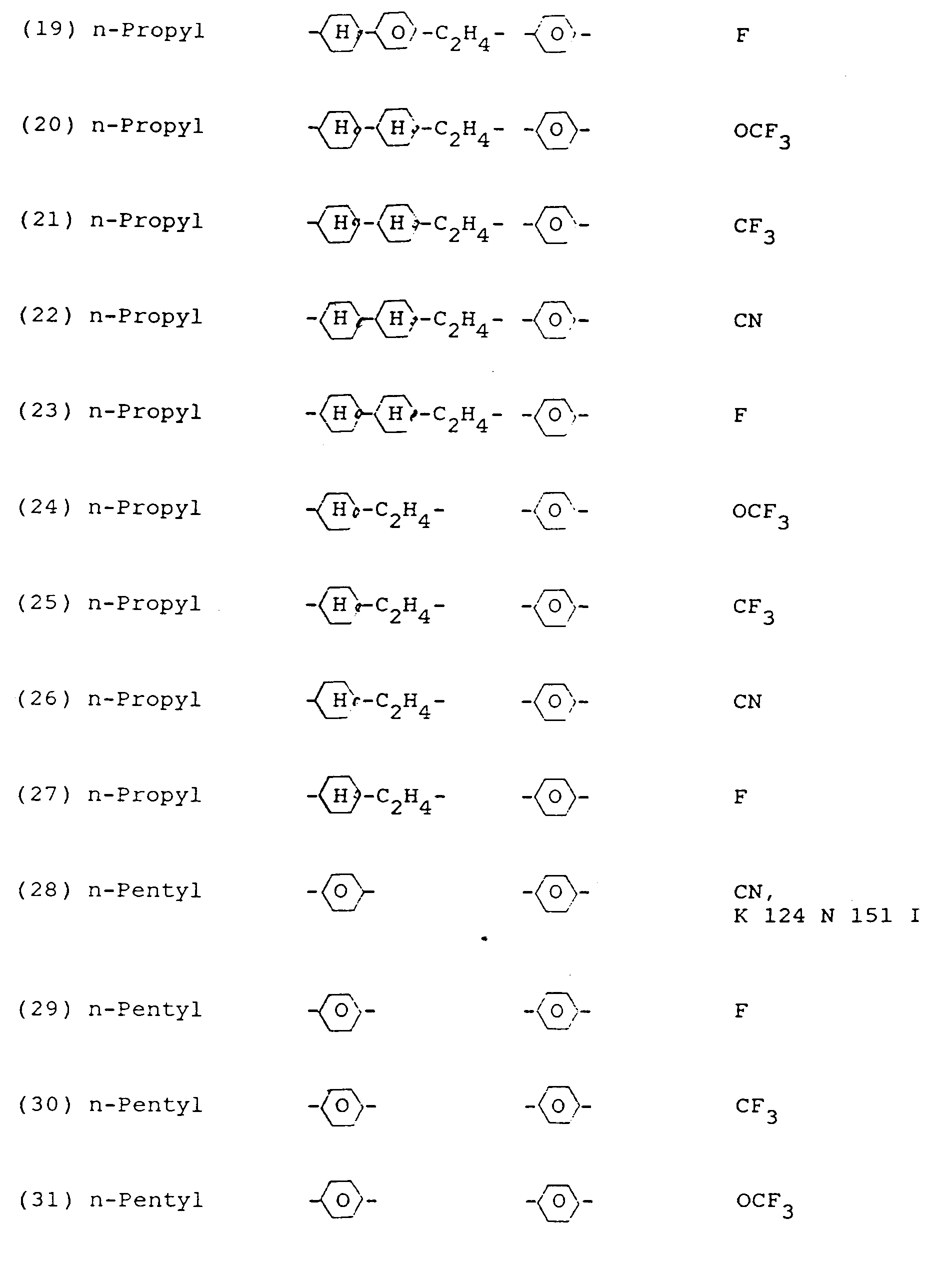

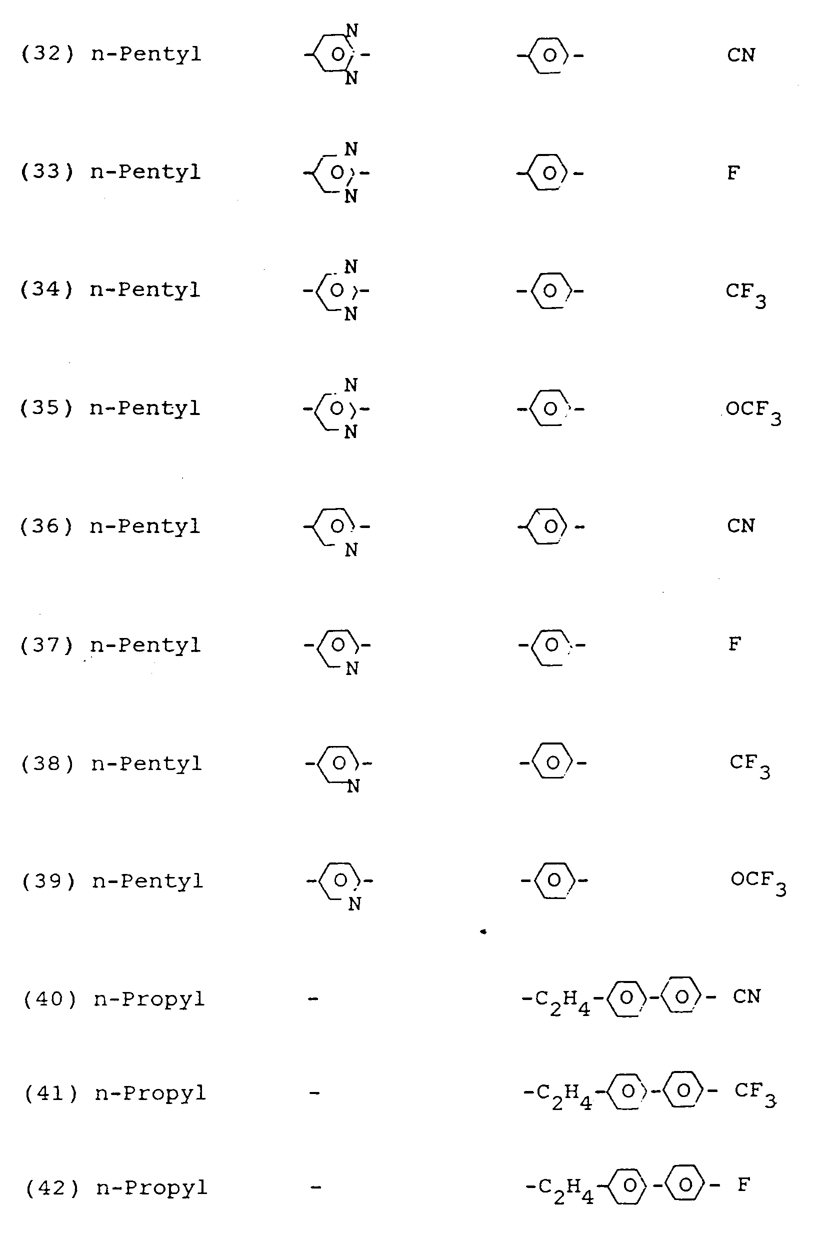

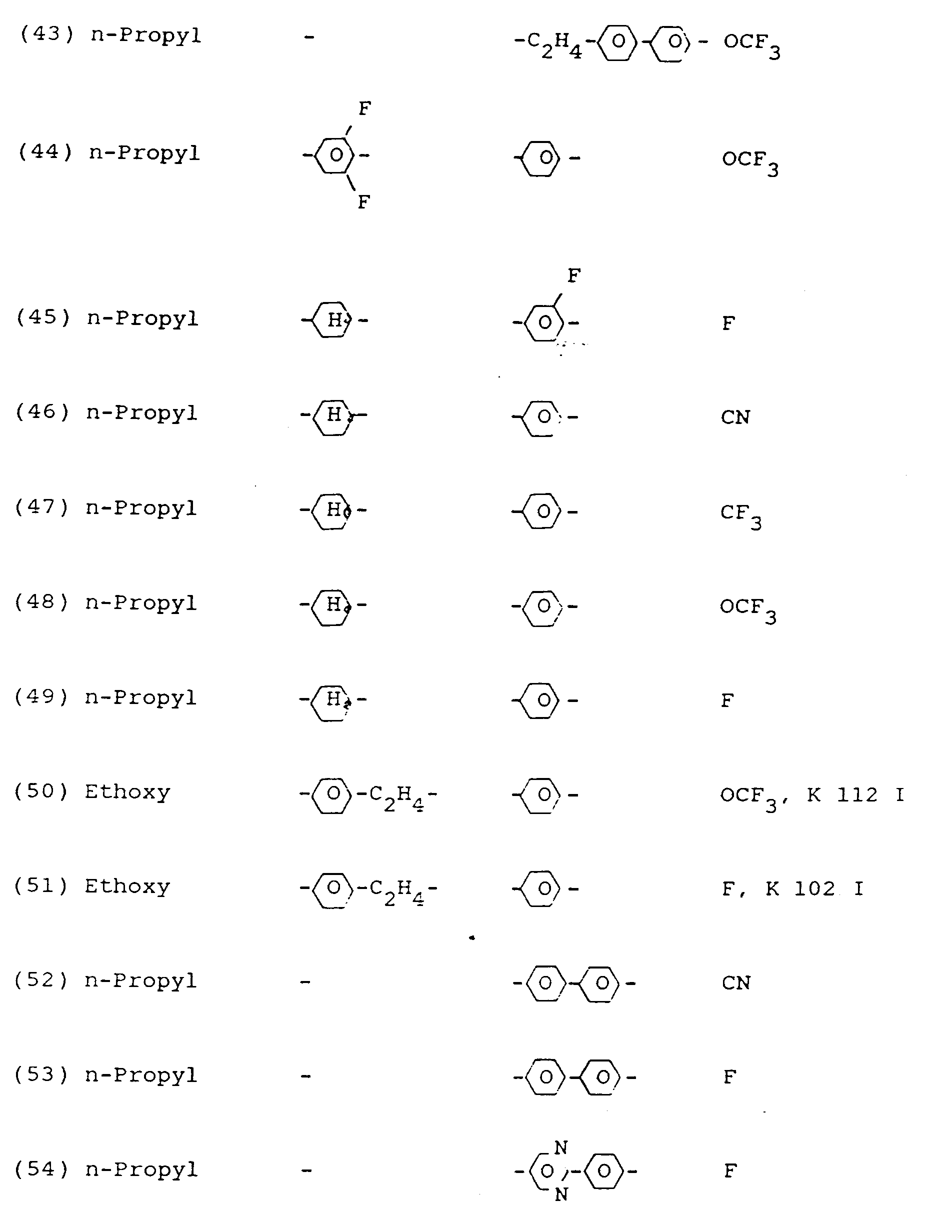

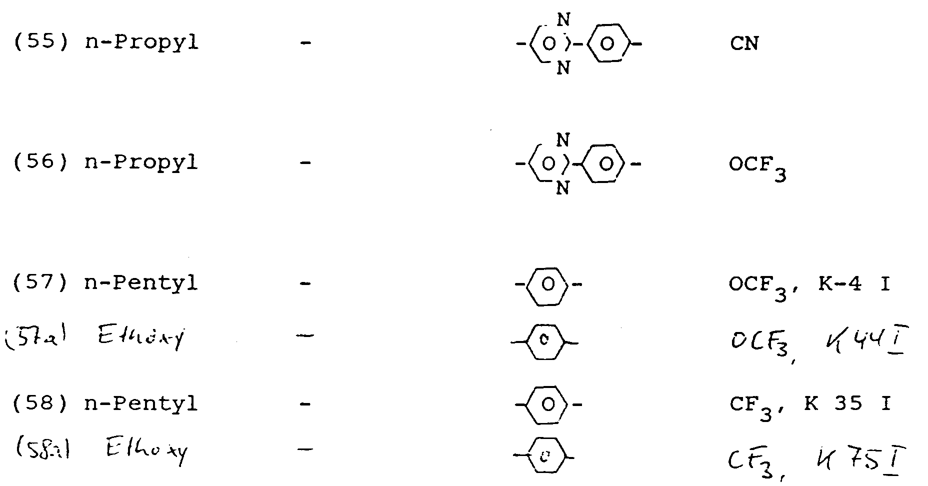

### Beispiel 59

Eine Lösung von 0,1 Mol p-Bromtrifluormethoxybenzol in 100 ml THF wird zu einer Suspension von 0,1 Mol Mg-Spänen in 50 ml THF so zugetropft, daß das Reaktionsgemisch gelinde siedet. Nach einer Stunde Rühren werden 0,1 Mol Trimethylborat bei Raumtemperatur zugetropft und nach einer weiteren Stunde Rühren mit verdünnter Salzsäure hydrolysiert. Nach extraktiver Aufarbeitung wird die erhaltene Boronsäure (0,05 Mol) zusammen mit 0,05 Mol 4-(trans-4-n-Pentylcyclohexyl)-2,6-difluorjodbenzol [erhältlich durch Umsetzung von 4-Pentylcyclohexanon mit 3,5-Difluorphenylmagnesiumbromid, Wasserabspaltung, und Hydrierung des Cyclohexenbenzols an Pd-C. Die erhaltene 3,5-Difluorpenylverbindung wird bei -70° in THF/TMEDA mit n-BuLi metalliert und anschließend mit Jod in THF bei -70° umgesetzt], 38 ml 2 mol wässeriger Na₂CO₃-Lsg. 0,6 g Tetrakis(triphenylphosphin)palladium-(O) und 75 ml Toluol zwei Stunden am Rückfluß gekocht. Nach üblicher wässeriger Aufarbeitung und chromatographischer Aufreinigung erhält man 4-(trans-4-n-Pentylcyclohexyl)-2,6-difluor-4'-trifluormethoxybiphenyl.

Analog erhält man aus den entsprechenden Difluorjodderivaten und Boronsäure die bevorzugten Verbindungen der Formel 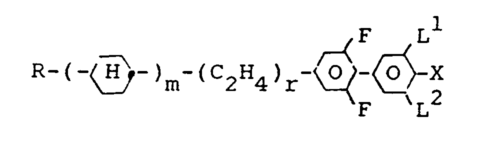 worin R, m und X die bei Formel I angegebene Bedeutung haben und r 0 oder 1 und L¹ und L² jeweils unäbhängig voneinander H oder F bedeuten.

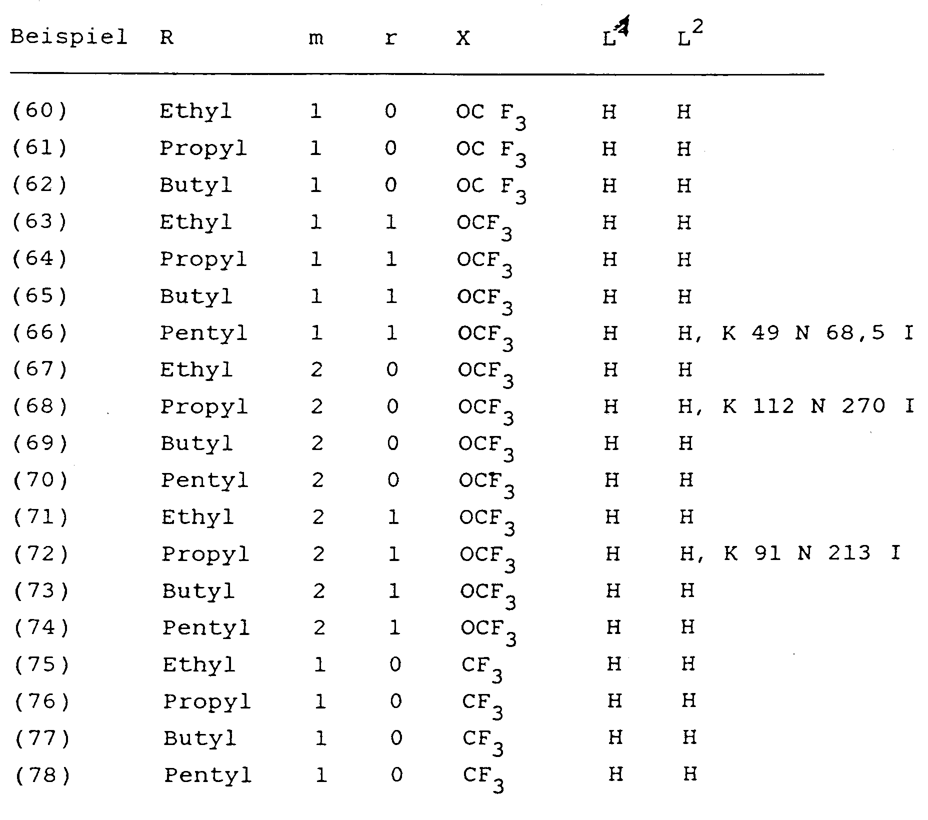

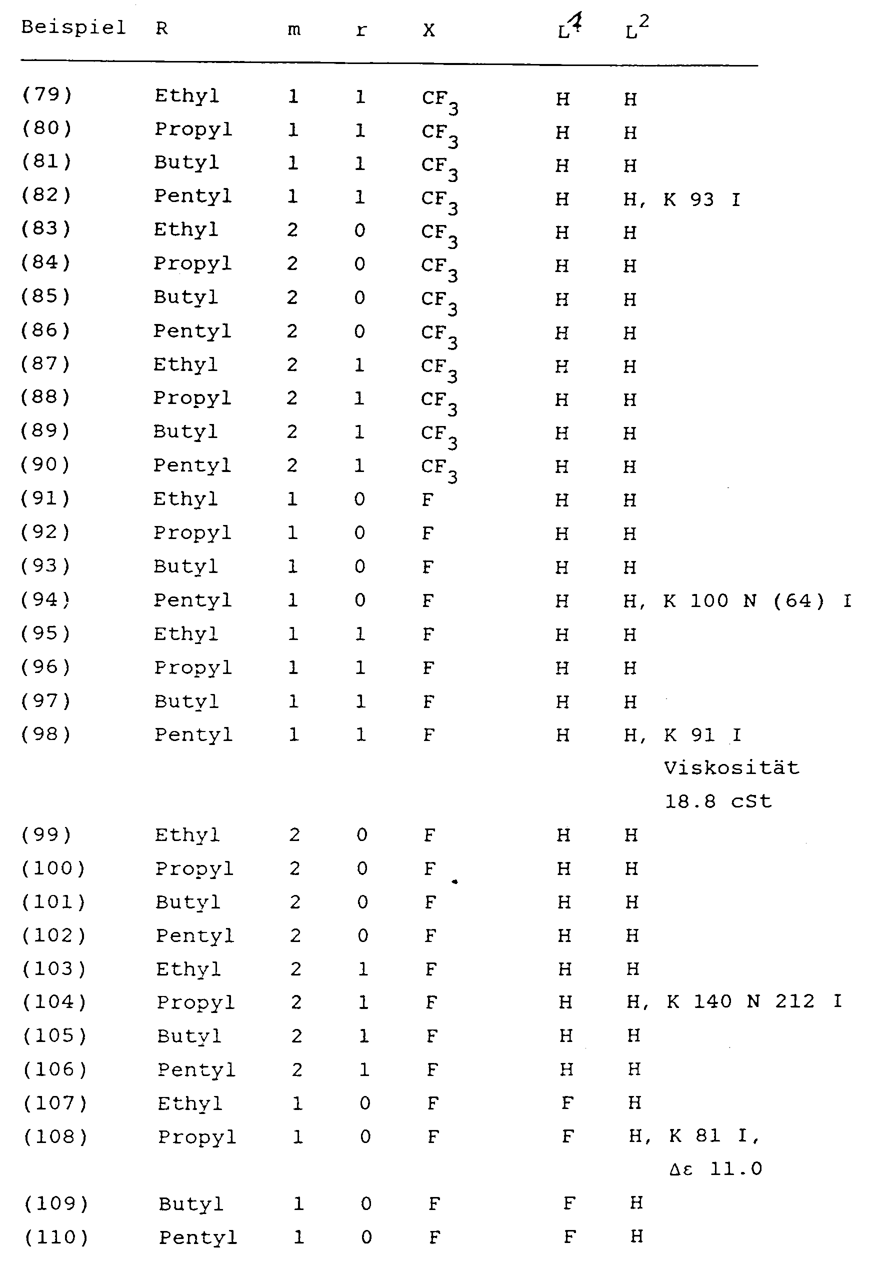

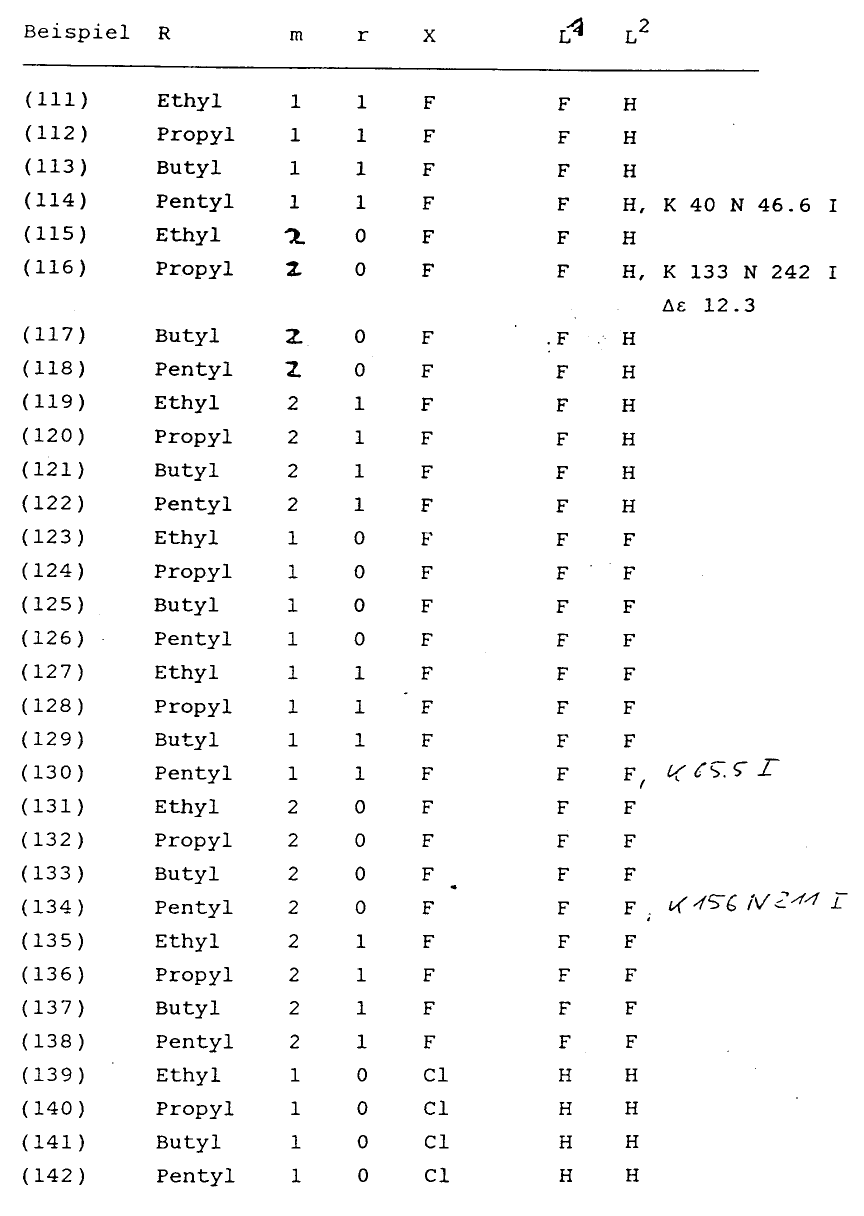

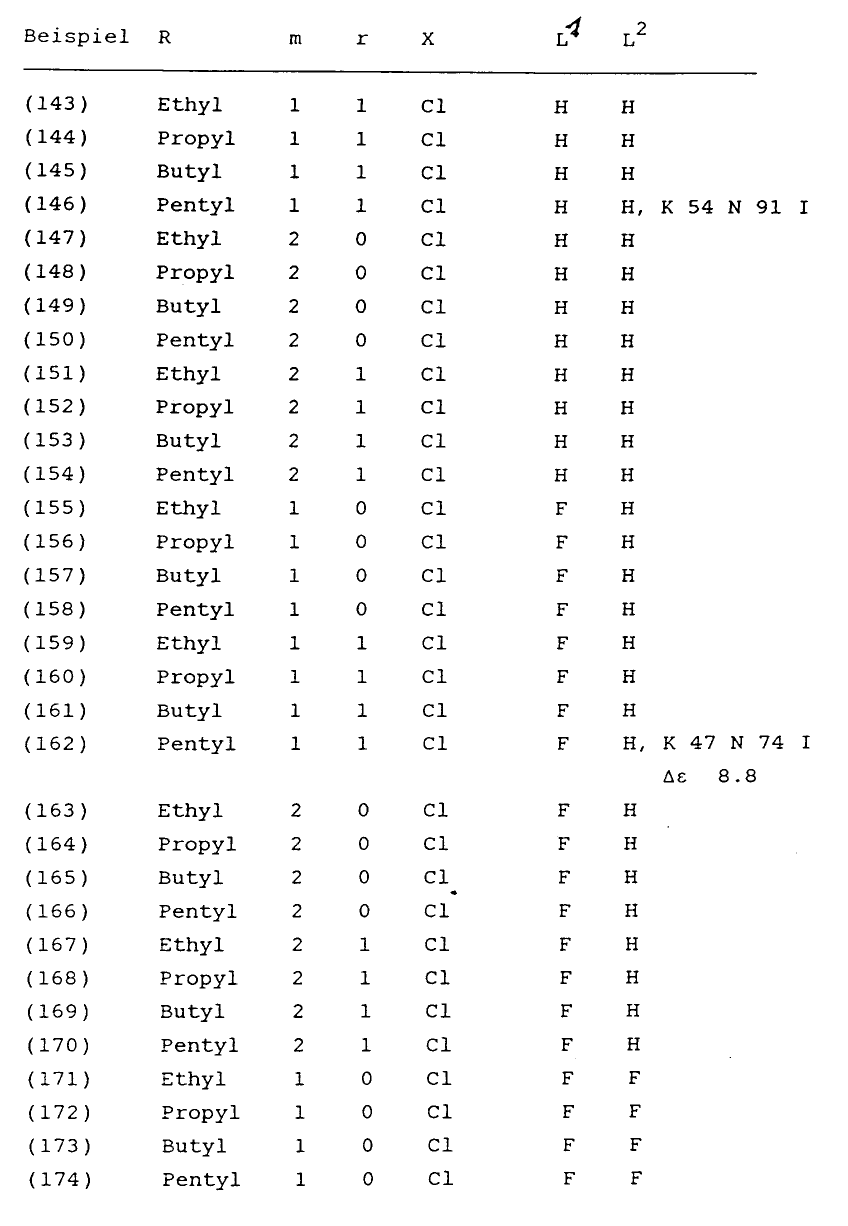

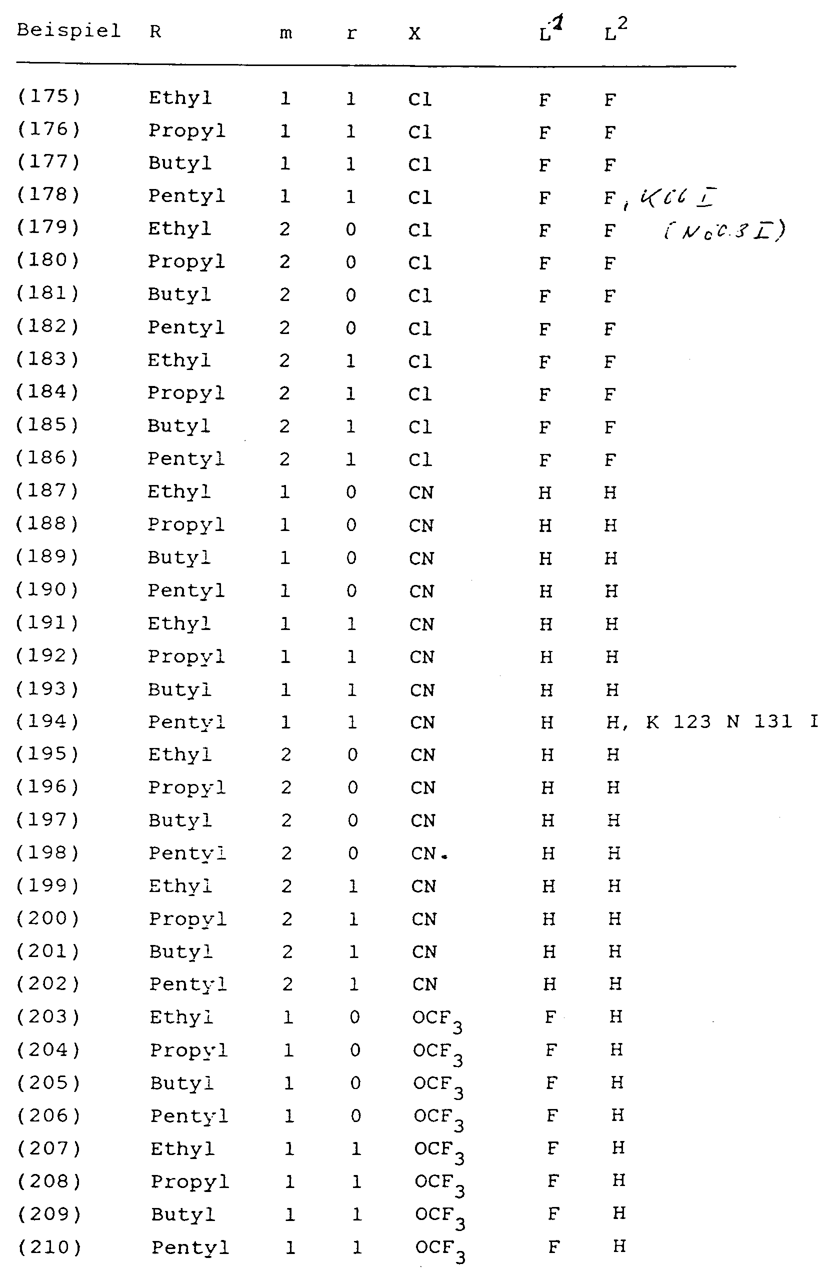

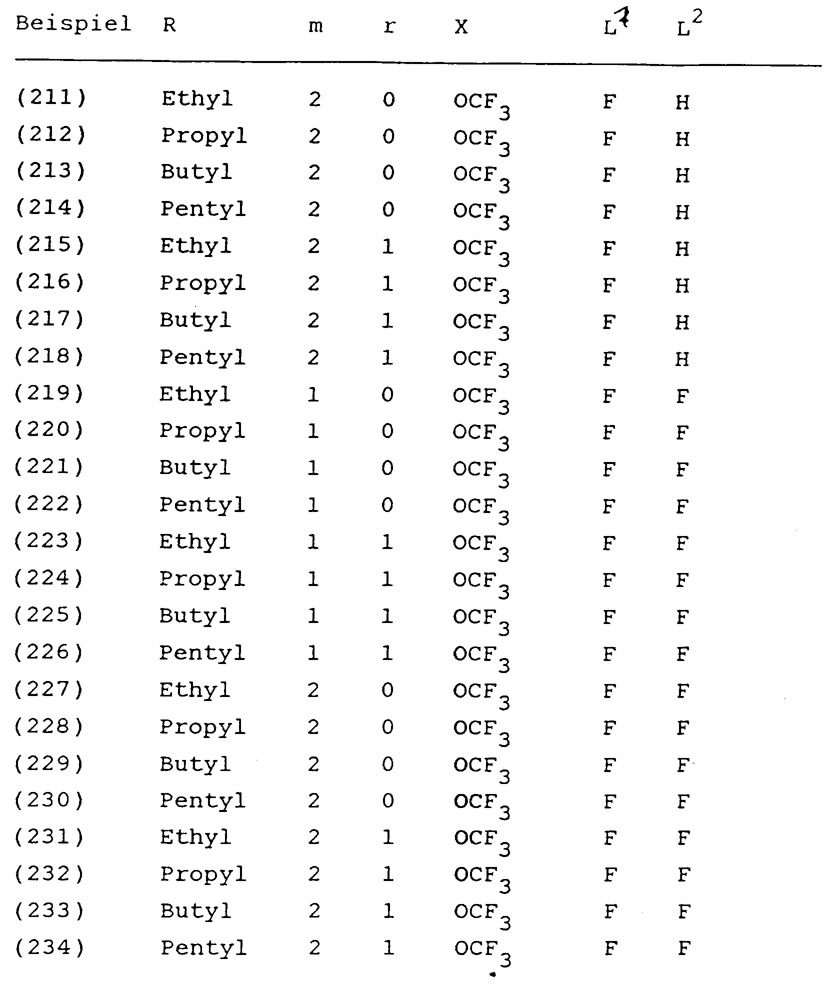

### Beispiel 235

Ein Gemisch von 9,2 g 2,6-Difluor-4-[2-(p-ethoxyphenyl)-ethyl]-phenylboronsäure (hergestellt durch Umsetzung von 3,5-Difluorbenzaldehyd mit p-Ethoxybenzyltriphenylphosphoniumjodid nach Wittig und nachfolgender Hydrierung des erhaltenen Styrolderivates an Pd/C), 6,0 g 1-Brom-4-n-propylbenzol, 38 ml 2 mol wässeriger Na₂CO₃-Lsg., 0,6 g Tetrakis(triphenylphosphin)palladium-(0) und 75 ml Toluol wird zwei Stunden am Rückfluß gekocht. Die wässerige Phase wird zweimal mit Toluol extrahiert und mit der org. Phase vereinigt. Nach üblicher wässeriger Aufarbeitung und chromatographischer Aufreinigung des Rückstandes erhält man 4-[2-(p-Ethoxyphenyl)-ethyl]-2,6-difluor-4'-n-propylbiphenyl.

### Beispiel 236 bis 242

Analog erhält man aus den entsprechenden Vorstufen der Formel II die folgenden erfindungsgemäßen Verbindungen:

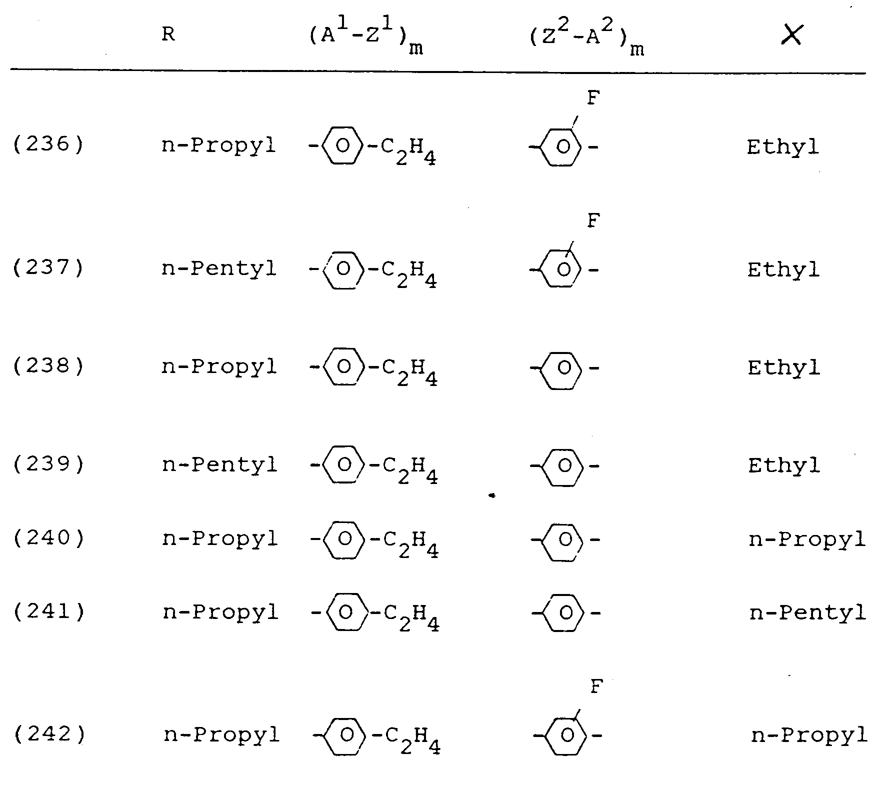

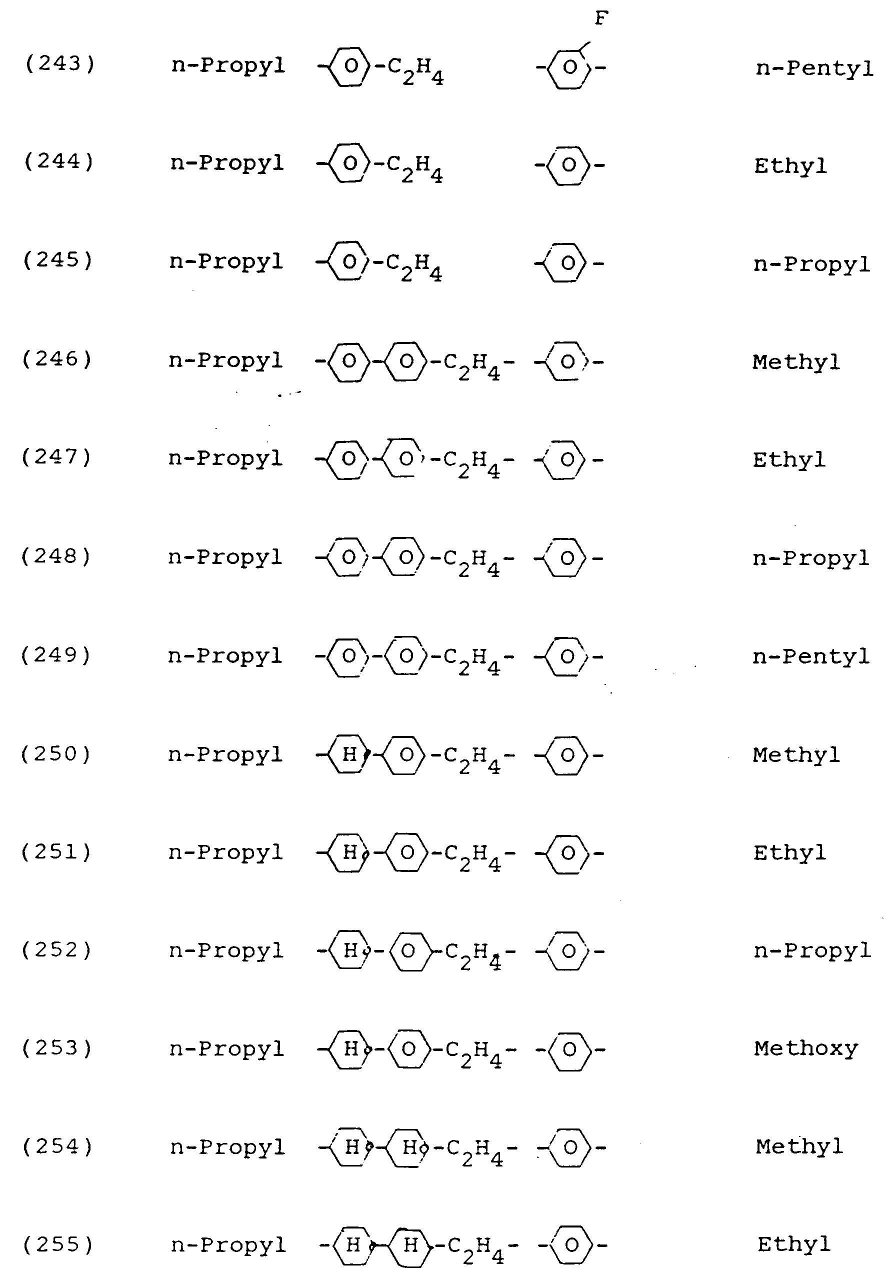

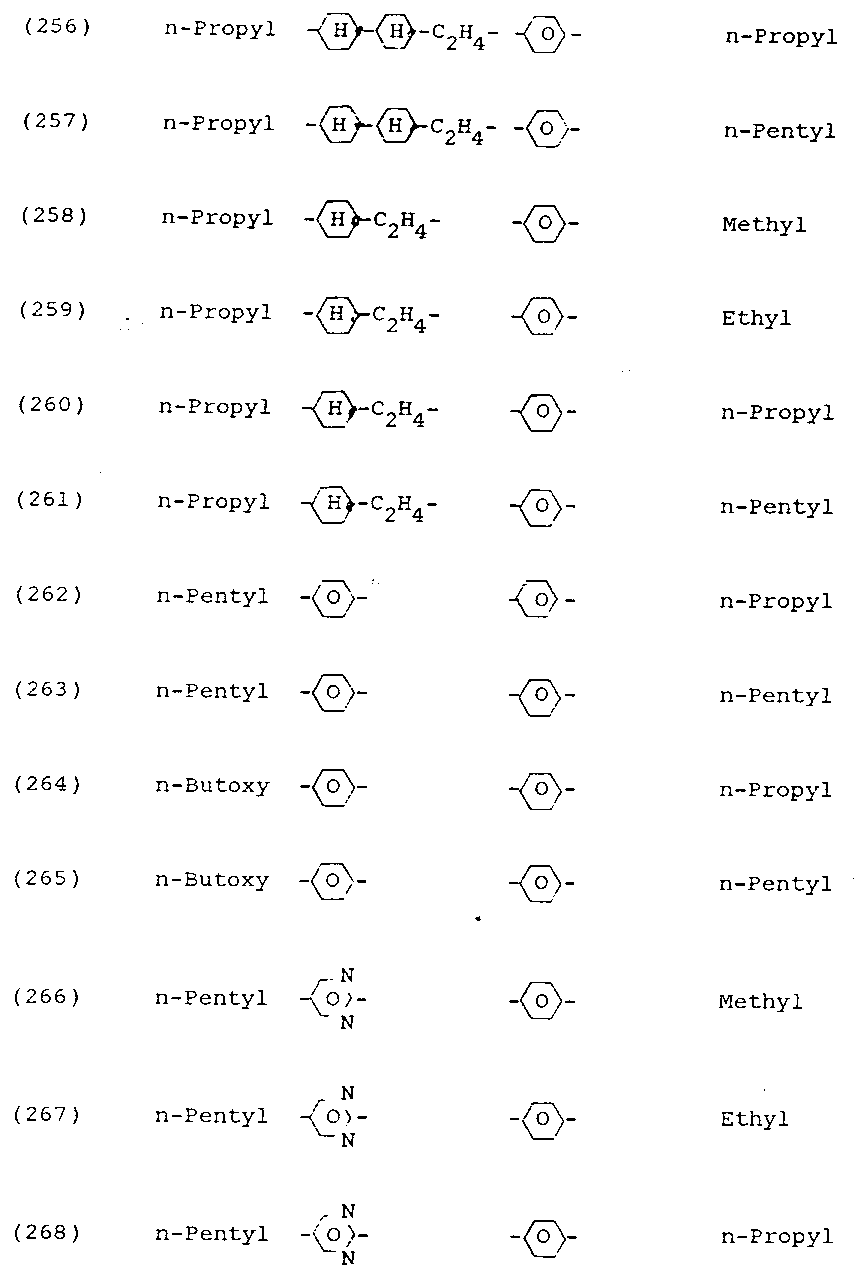

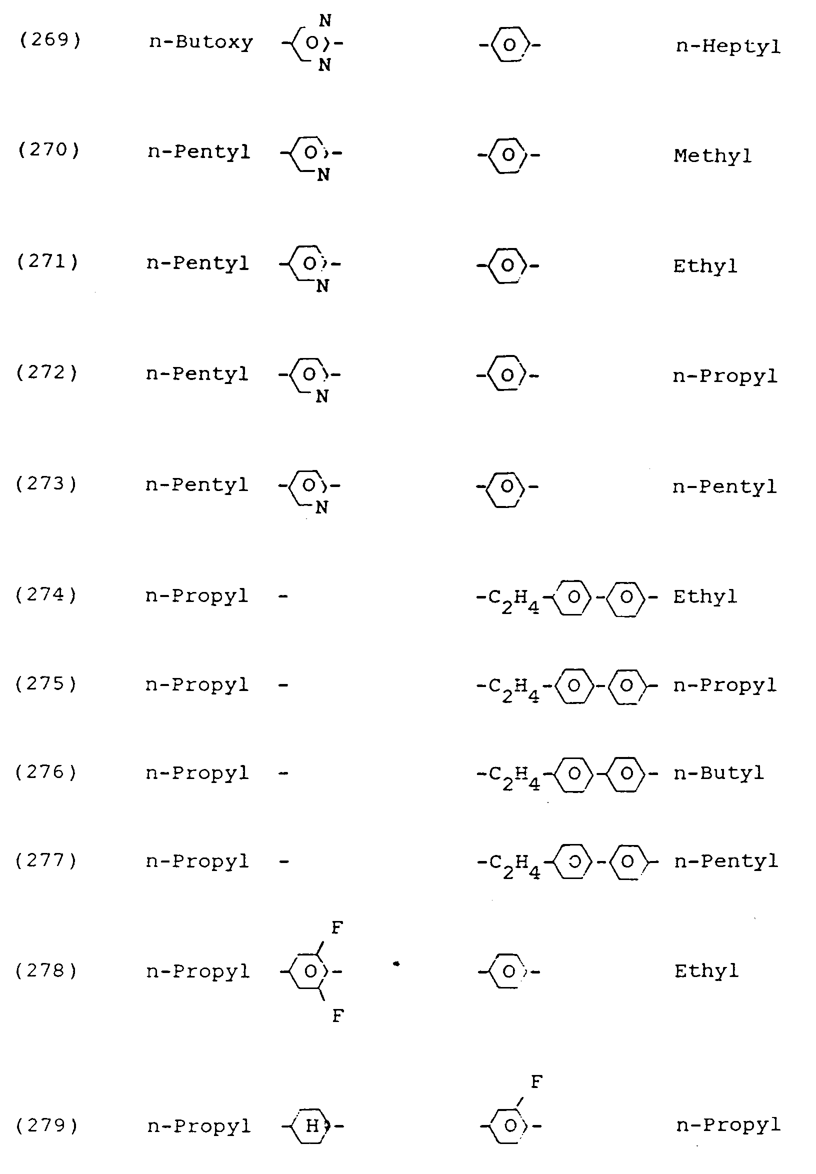

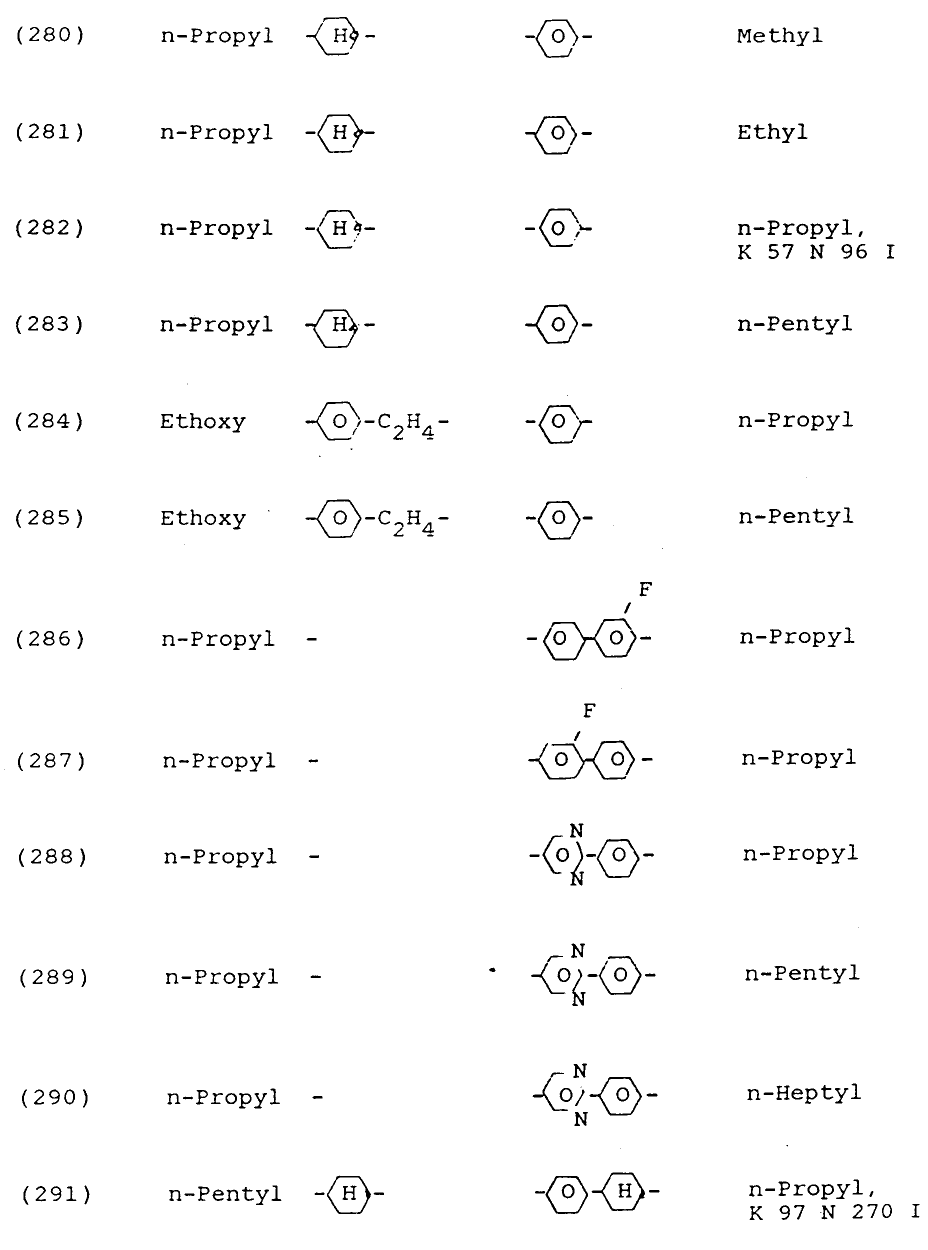

### Beispiel 292

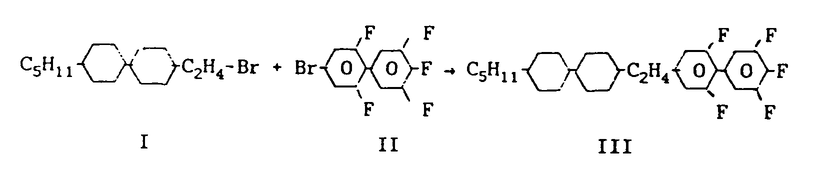

0,1 Mol I werden in 150 ml eines Lösungsmittelgemisches von THF/Toluol (1:4-Volumenverhältnis) vorgelegt, dann 11,5 g Zinkbromid wasserfrei und darauf 1,4 g Lithiumgranulat hinzugefügt. Das Gemisch wird unter Argon und Rühren 4 Std. zwischen 0 °C und 10 °C im Ultraschall behandelt, um I in die entsprechende Dialkylzinkverbindung zu überführen. Die zinkorganische Verbindung wird mit 0,1 Mol II und 1,5 g (2 mol %) 1,1'-Bis(diphenylphosphino)-ferrocen-palladium-(II)-dichlorid (PdCl₂ dppf) versetzt und nach Entfernung des Ultraschallbades und der Kühlung 24 h bei Zimmertemperatur gerührt. Es wird mit 100 ml gesättigter NH₄Cl-Lösung unter Rühren zersetzt, die organische Phase abgetrennt, die wäßrige Phase 2 x mit Toluol extrahiert. Die vereinigten organischen Extrakte liefern nach dem Trocknen, Einengen und Chromatographieren über Kieselgel mit Hexan III.

### Beispiel 293

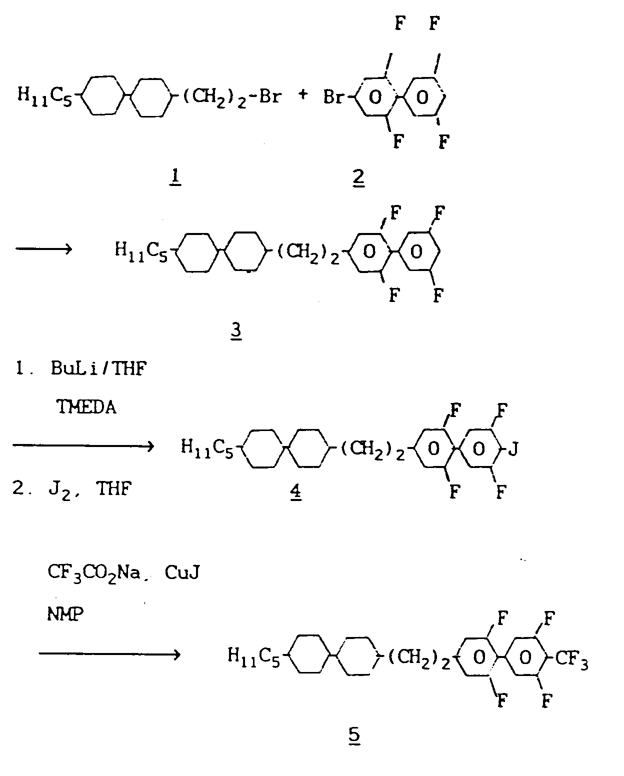

100 mmol 1 werden analog zum vorstehenden Beispiel durch Umsetzung mit 2 in 3 überführt.

Zu einem Gemisch aus 47 mmol 3, 7,5 ml TMEDA (50 mmol) und 150 ml THF werden bei -65 bis -70 °C 31 ml n-BuLi (15 % in Hexan) zugetropft und es wird 1 Stunde bei -70 °C nachgerührt. Dann wird bei -65 bis -70 °C eine Lösung von 12,0 g (47 mmol) Jod in 25 ml THF zugetropft und 0,5 h bei -70 °C nachgerührt. Man erwärmt auf -30 °C, hydrolysiert mit 15 ml Wasser und reduziert überschüssiges Jod durch Zusatz von 15 ml Natriumhydrogensulfitlösung. Nach üblicher Aufarbeitung und Umkristallisation aus Hexan erhält man 4. Aus einem Gemisch von 38 mmol 4, 4,4 g (76 mmol) KF, 22,8 g (168 mmol) Natriumtrifluoracetat und 800 ml NMP werden bei 70 °C und 4 mbar 400 ml NMP abdestilliert. Dann gibt man 1,4 g (76 mmol) getrocknetes CuJ zum Reaktionsgemisch und rührt5 h bei 160 °C. Anschließend werden ca. 300 ml NMP abdestilliert. Man läßt auf RT abkühlen und versetzt mit 400 ml MTB-Ether. Man wäscht mit Wasser, trocknet mit Na₂SO₄, filtriert und engt zum Rücktand ein. Nach Chromatographie an Kieselgel mit Hexan erhält man 5.

### Beispiel 294

Eine Lösung von 0,1 m 1-(trans-4-n-Propylcyclohexyl)-2-(3,3',5'-trifluorbiphenylyl)-ethan und 0,1 m TMEDA in 300 ml THF wird bei ca. -65° mit 0,1 m n-BuLi (1,5 M in Hexan) tropfenweise versetzt. Man rührt noch 30 Min. bei dieser Temperatur und setzt dann 0,2 m N-Chlorsuccinimid in 70 ml THF langsam zu. Nach beendeter Zugabe läßt man auf -20° erwärmen und hydrolysiert mit H₂O. Durch Zugabe von Diethylether wird das Produkt vollständig in Lösung gebracht. Nach extraktiver Aufarbeitung und Reinigung durch Chromatographie und Kristallisation erhält man 1-(trans-4-n-Propylcyclohexyl)-2-(4'-chlor-3,3',5'-trifluorbiphenylyl)-ethan.

### Beispiel 301

Ein Gemisch von 10,5 g 1-Brom-3,4-difluorbenzol, 17,4 g 4-(trans-4-4-Propylcyclohexyl)-2,6-difluorstyrol (erhältlich nach den o.a. Schemata), 0,25 g Pd-acetat, 0,6 g Tri-o-tolylphosphin, 7 g Triethylamin und 125 ml Acetonitril wird am Rückfluß erhitzt bis zur Beendigung der Reaktion. Nach üblicher Aufarbeitung erhält man trans-1-(3,4-Difluorphenyl)-2-[4-(trans-4-Propylcyclohexyl)-2,6-difluorphenyl]-ethen, K 101 N 156.5 I.

Analog wird hergestellt:
trans-1-(3,4-Difluorphenyl)-2-(2,6-Difluor-4-ethoxyphenyl)-ethen, K 92 I

### Beispiel A

Ein flüssigkristallines Medium I besteht aus

| | |
|---|---|
| PCH-5F | 10,0 % |
| PCH-6F | 8,0 % |
| PCH-7F | 6,0 % |
| CCP-20CF3 | 8,0 % |
| CCP-30CF3 | 12,0 % |
| CCP-40CF3 | 9,0 % |
| CCP-50CF3 | 9,0 % |
| BCH-3F.F | 12,0 % |
| BCH-5F.F | 10,0 % |
| ECCP-30CF3 | 5,0 % |
| ECCP-50CF3 | 5,0 % |
| CBC-33F | 2,0 % |
| CBC-53F | 2,0 % |
| CBC-55F | 2,0 % |

Ein erfindungsgemäßes Medium II besteht aus 90 % Medium I und 10 %

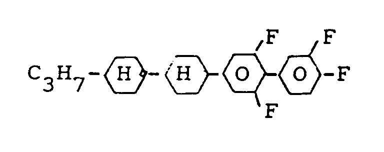

| | Medium I | Medium II |
|---|---|---|
| Klärpunkt | 91 °C | 102,4 °C |
| Δn | 0,094 | 0,102 |
| Δε | 5,2 | 6,0 |
| Viskosität bei 20 ° (cSt) | 15,0 | 16,7 |

## Patentansprüche

1. 1,4-Disubstituierte 2,6-Difluorbenzolverbindungen der Formel I, 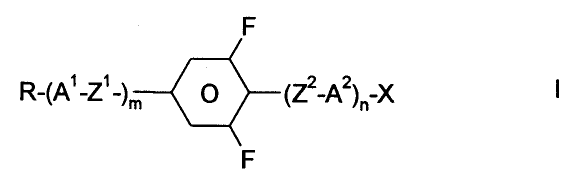 worin
R einen unsubstituierten oder einen mindestens einfach durch Halogen substituierten Alkyl- oder Alkenylrest mit 1 bis 15 C-Atomen, wobei in diesen Resten auch eine oder mehrere CH₂-Gruppen jeweils unabhängig voneinander durch -O-, -S-, 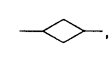 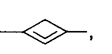 -CO-, -CO-O-, -O-CO-oder -O-CO-O-so ersetzt sein können, daß O-Atome nicht direkt miteinander verknüpft sind,
A¹ und A² jeweils unabhängig voneinander einen
(a) trans-1,4-Cyclohexylenrest, worin auch eine oder mehrere nicht benachbarte CH₂-Gruppen durch -O- und/oder -S- ersetzt sein können,
(b) 1,4-Phenylenrest, worin auch eine oder zwei CH-Gruppen durch N ersetzt sein können,
(c) Rest aus der Gruppe 1,4-Cyclohexylen, 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) und (b) mit CN oder ein- oder zweifach mit Fluor substituiert sein können.
Z¹ und Z² jeweils unabhängig voneinander -CO-O-, -O-CO-, -CH₂O-, -OCH₂-, -CH₂CH₂-, -CH=CH-, -C≡C- oder eine Einfachbindung,
m 0, 1, 2 oder 3,
n 1, 2 oder 3, und
X F, Cl, -CF₃, -OCF₃-, -OCF₂H, -OC₂F₅ oder -NCS bedeutet,
mit der Maßgabe, daß
mindestens einer der im Molekül vorhandenen Reste Z¹ und Z² -CO-O-bedeutet.

2. Verwendung von Verbindungen der Formel I als Komponenten flüssigkristalliner Medien.

3. Flüssigkristallines Medium mit mindestens zwei flüssigkristallinen Komponenten, dadurch gekennzeichnet, daß sie mindestens eine Verbindung der Formel I enthält.

4. Flüssigkristall-Anzeigeelement, dadurch gekennzeichnet, daß es ein flüssigkristallines Medium nach Anspruch 3 enthält.

5. Elektrooptische Anzeige, dadurch gekennzeichnet, daß sie als Dielektrikum ein flüssigkristallines Medium nach Anspruch 3 enthält.

## Claims

1. 1,4-Disubstituted 2,6-difluorobenzene compounds of the formula I 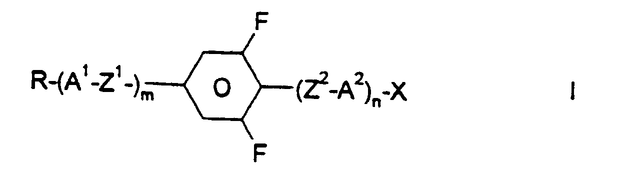 where
R is an alkyl or alkenyl radical having 1 to 15 carbon atoms which is unsubstituted or at least monosubstituted by halogen, it also being possible for one or more CH₂ groups in these radicals to be replaced, in each case independently of one another, by -O-, -S-, 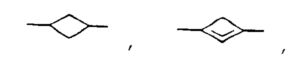 -CO-, -CO-O-, -O-CO- or -O-CO-O- in such a manner that oxygen atoms are not linked directly to one another,
A¹ and A², in each case independently of one another, are
(a) a trans-1,4-cyclohexylene radical in which, in addition, one or more non-adjacent CH₂ groups may be replaced by -O-and/or -S-,
(b) a 1,4-phenylene radical in which, in addition, one or two CH groups may be replaced by N,
(c) a radical from the group comprising 1,4-Cyclohexenylene, 1,4-bicyclo(2,2,2)-octylene, piperidine-1,4-diyl, naphthalene-2,6-diyl, decahydronaphthalene-2,6-diyl and 1,2,3,4-tetrahydronaphthalene-2,6-diyl,
it being possible for the radicals (a) and (b) to be substituted by CN or monosubstituted or disubstituted by fluorine,
Z¹ and Z², in each case independently of one another, are -CO-O-, -O-CO-, -CH₂O-, -OCH₂-, -CH₂CH₂-, -CH=CH-, -C≡C- or a single bond,
m is 0, 1, 2 or 3,
n is 1, 2 or 3, and
X is F, Cl, -CF₃, -OCF₃ , -OCF₂H, -OC₂F₅ or -NCS, with the proviso that
at least one of the radicals Z¹ and Z² present in the molecule is -CO-O-.

2. Use of compounds of the formula I as components of liquid-crystalline media.

3. Liquid-crystalline medium containing at least two liquid-crystalline components, characterized in that it contains at least one compound of the formula I.

4. Liquid-crystal display element, characterized in that it contains a liquid-crystalline medium according to Claim 3.

5. Electrooptical display, characterized in that it contains, as dielectric, a liquid-crystalline medium according to Claim 3.

## Revendications

1. Les composés 2,6-difluorobenzène disubstitués en 1,4 de formule I 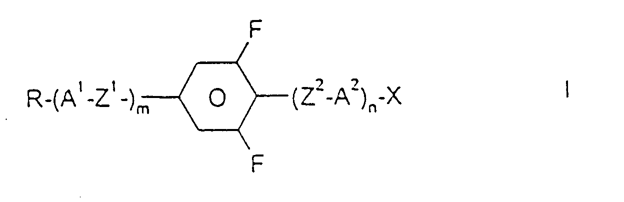 où
R représente un reste alkyle ou alcényle comportant 1 à 15 atomes de C, non substitué ou au moins monosubstitué par un halogène, un ou plusieurs groupes CH₂ dans ces restes pouvant être également remplacés, indépendamment les uns des autres, par -0-, -S-, 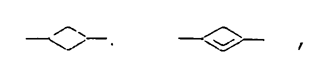 -CO-, -CO-O-, -O-CO- ou -O-CO-O- de telle façon que les atomes d'oxygène ne soient pas directement reliés entre eux,
A¹ et A² représentent, indépendamment l'un de l'autre,
(a) un reste trans-1,4-cyclohexylène dans lequel un ou plusieurs groupes CH₂ non adjacents peuvent être également remplacés par -O- et/ou -S-,
(b) un reste 1,4-phénylène dans lequel un ou deux groupes CH peuvent être remplacés par N,
(c) un reste choisi dans le groupe constitué par le 1,4-cyclohexenylène,le 1,4-bicyclo(2,2,2)octylène, le pipéridine-1,4-diyle, le naphtalène-2,6-diyle, le décahydronaphtalène-2,6-diyle et le 1,2,3,4-tétrahydronaphtalène-2,6-diyle,
les restes (a) et (b) pouvant être substitués par CN ou mono- ou disubstitués par le fluor,
Z¹ et Z² représentent, indépendamment l'un de l'autre, -CO-O-, -O-CO-, -CH₂O-, -OCH₂-, -CH₂CH₂-, -CH=CH-, -C≡C-ou une liaison simple,
m est égal à O, 1, 2 ou 3,
n est égal à 1, 2 ou 3 et
X représente F, Cl, -CF₃, -OCF₃ , -OCF₂H, -OC₂F₅ ou -NCS,
sous réserve
qu'au moins l'un des restes Z¹ et Z² présent dans la molécule représente -CO-O-.

2. Utilisation des composés de formule I comme composants de milieux à cristaux liquides.

3. Milieu à cristaux liquides comportant au moins deux composants à cristaux liquides, caractérisé en ce qu'il contient au moins un composé de formule I.

4. Elément d'affichage à cristaux liquides, caractérisé en ce qu'il contient un milieu à cristaux liquides selon la revendication 3.

5. Affichage électro-optique caractérisé en ce qu'il contient un milieu à cristaux liquides selon la revendication 3 comme diélectrique.
